(19)

Europäisches
Patentamt
European
Patent Office
Office européen
des brevets

(11)     EP 2 511 843 B1

(12)          **EUROPEAN PATENT SPECIFICATION**

(45) Date of publication and mention
of the grant of the patent:
**21.12.2016 Bulletin 2016/51**

(51) Int Cl.:
*G06F 19/18* (2011.01)          *G06F 19/22* (2011.01)

(21) Application number: **12165247.3**

(22) Date of filing: **28.04.2010**

(54) **Method and system for calling variations in a sample polynucleotide sequence with respect to a reference polynucleotide sequence**

Verfahren und System zum Aufrufen von Variationen in einer Polynukleotidprobensequenz in Bezug auf eine Referenzpolynukleotidsequenz

Procédé et système pour appeler des variations dans une séquence polynucléotidique d'échantillon par rapport à une séquence polynucléotidique de référence

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **29.04.2009 US 173967 P**

(43) Date of publication of application:
**17.10.2012 Bulletin 2012/42**

(62) Document number(s) of the earlier application(s) in accordance with Art. 76 EPC:
**10770290.4 / 2 430 441**

(73) Proprietor: **Complete Genomics, Inc.
San Francisco, CA 94043 (US)**

(72) Inventors:
• **Carnevali, Paolo
San Francisco, CA California 94043 (US)**
• **Baccash, Jonathan, M.
San Francisco, CA California 94043 (US)**
• **Nazarenko,, Igor
San Francisco, CA California 94043 (US)**
• **Halpern, Aaron, L.
San Francisco, CA California 94043 (US)**
• **Nilsen Geoffrey
San Francisco, CA California 94043 (US)**
• **Martin, Bruce
San Francisco, CA California 94043 (US)**
• **Drmanac, Radoje
San Francisco, CA California 94043 (US)**

(74) Representative: **Eisenführ Speiser
Patentanwälte Rechtsanwälte PartGmbB
Johannes-Brahms-Platz 1
20355 Hamburg (DE)**

(56) References cited:
• **LI HENG ET AL: "MAPPING SHORT DNA SEQUENCING READS AND CALLING VARIANTS USING MAPPING QUALITY SCORES", GENOME RESEARCH, COLD SPRING HARBOR LABORATORY PRESS, WOODBURY, NY, US, vol. 18, 1 January 2008 (2008-01-01), pages 1851-1858, XP001503357, ISSN: 1088-9051, DOI: 10.1101/GR.078212.108 [retrieved on 2008-08-19]**
• **MARTH G T ET AL: "GENERAL APPROACH TO SINGLE-NUCLEOTIDE POLYMORPHISM DISCOVERY", NATURE GENETICS, NATURE PUBLISHING GROUP, NEW YORK, US, vol. 23, no. 4, 1 December 1999 (1999-12-01), pages 452-456, XP000920898, ISSN: 1061-4036, DOI: 10.1038/70570**
• **ZERBINO D R ET AL: "Velvet: algorithms for de novo short read assembly using de Bruijn graphs", GENOME RESEARCH, COLD SPRING HARBOR LABORATORY PRESS, WOODBURY, NY, US, vol. 18, no. 5, 1 May 2008 (2008-05-01), pages 821-829, XP008096312, ISSN: 1088-9051, DOI: 10.1101/GR.074492.107**

- ANNEXSTEIN F S: "Generating De Bruijn sequences: an efficient implementation", IEEE TRANSACTIONS ON COMPUTERS, IEEE SERVICE CENTER, LOS ALAMITOS, CA, US, vol. 46, no. 2, 1 February 1997 (1997-02-01), pages 198-200, XP008148192, ISSN: 0018-9340, DOI: 10.1109/12.565596

- CHAN A H ET AL: "On the Complexities of de Bruijn Sequences", JOURNAL OF COMBINATORIAL THEORY. SERIES A, ACADEMIC PRESS, INC., LONDON, GB, vol. 33, no. 3, 1 November 1982 (1982-11-01), pages 233-246, XP008148194, ISSN: 0097-3165, DOI: 10.1016/0097-3165(82)90038-3 [retrieved on 2004-09-02]

SEQUENCE LISTING

<110> Complete Genomics, Inc.

<120> Method and system for calling variations in a sample polynucleotide sequence with respect to a reference polynucleotide sequence

<140> EP12165247.3-1225
<141> 2012-04-24

<150> US61/173,967
<151> 2009-04-29

<160> 4

<170> BiSSAP 1.0

<210> 1
<211> 25
<212> DNA
<213> Homo sapiens

<220>
<221> source
<222> 1..25
<223> /mol_type="DNA"
/organism="Homo sapiens"

<400> 1
acgatacgag tagaaaaaaa ctata          25

<210> 2
<211> 24
<212> DNA
<213> Homo sapiens

<220>
<221> source
<222> 1..24
<223> /mol_type="DNA"
/organism="Homo sapiens"

<400> 2
acgatacgag tagaaaaaac tata          24

<210> 3
<211> 24
<212> DNA
<213> Homo sapiens

<220>
<221> source
<222> 1..24
<223> /mol_type="DNA"
/organism="Homo sapiens"

<400> 3
actatacgag tagaaaaaac tata          24

<210> 4

<211> 18
<212> DNA
<213> Homo sapiens

<220>
<221> source
<222> 1..18
<223> /mol_type="DNA"
/organism="Homo sapiens"

<400> 4
ctacgatacg acacgact        18

**Description**

BACKGROUND OF THE INVENTION

In the following discussion certain articles and methods will be described for background and introductory purposes. Nothing contained herein is to be construed as an "admission" of prior art. Applicant expressly reserves the right to demonstrate, where appropriate, that the articles and methods referenced herein do not constitute prior art under the applicable statutory provisions.

Genetic studies have seen rapid advances in recent years. The entire genomes of specific organisms, including some individual human beings, have been sequenced and become available as references, i.e., genetic sequences used as references for studying members of the same species. In genetic research, genetic testing, personalized medicine, and many other applications, it is often useful to obtain a sample of genetic material, determine a sequence of that sample, and to analyze that sequence with respect to one or more existing references to identify sequence variations or obtain other useful information for the sample.

Existing approaches to genetic testing typically locate or map long, contiguous sample sequences to positions in a reference. However, some techniques used for obtaining sample sequences yield a polynucleotide sequence comprising multiple shorter sequences (sometimes referred to as oligomers) with predicted spatial relationships, and in some cases with spatial relationships of variable distances. In the latter case, the relative genomic positions of bases in these shorter sequences are only approximately known, and are generally in the form of short contiguous reads with variable but constrained amounts of spacing or overlap (referred to as gap distance).

Conventional techniques available for sequence assembly are not adequate to provide high speed, low cost de novo assembly or reassembly of short sequences comprising contiguous reads that are variably gapped. Accordingly, there is a need for improved methods and systems for variation calling and assembly. The present invention addresses this need.

Heng L. et al., "Mapping short DNA sequencing reads and calling variants using mapping quality scores", Genome Research, Vol. 18, No. 11, 2008, pages 1851-1858 describes a mapping quality that measures the confidence that a read actually comes from the position it is aligned to by the mapping algorithm. The position with the highest mapping quality is chosen as the true alignment. A position will be chosen randomly if a read can be mapped equally to multiple positions. The consensus sequence at a position is determined by calculating a probability of a genotype given observing data D.

Marth G. T. et al., "A general approach to single-nucleotide polymorphism discovery", Nature Genetics, Vol. 23, No. 4, 1999, pages 452-456 aligns ESTs. A Bayesian model is then used to calculate a probability that a site is polymorphic.

SUMMARY OF THE INVENTION

This Summary is provided to introduce a selection of concepts in a simplified form that are further described below in the Detailed Description. This Summary is not intended to identify key or essential features of the claimed subject matter, nor is it intended to be used to limit the scope of the claimed subject matter. Other features, details, utilities, and advantages of the claimed subject matter will be apparent from the following written Detailed Description including those aspects defined in the appended claims.

Exemplary embodiments provide methods and systems for calling variations in a sample polynucleotide sequence compared to a reference polynucleotide sequence. Mapped mated reads can include both arms aligned to a reference, or to one arm aligned to a reference. Aspects of the embodiments include executing an application on at least one computer that locates local areas in the reference polynucleotide sequence where a likelihood exists that one or more bases of the sample polynucleotide sequence are changed from corresponding bases in the reference polynucleotide sequence, where the likelihood is determined at least in part based on mapped mated reads of the sample polynucleotide sequence; generating at least one sequence hypothesis for each of the local areas, and optimizing the at least one sequence hypothesis for at least a portion of the local areas to find one or more optimized sequence hypotheses of high probability for the local areas; and analyzing the optimized sequence hypotheses to identify a series of variation calls in the sample polynucleotide sequence. According to the exemplary embodiments, the variation calls made in the sample polynucleotide sequence are strongly supported by the data.

According to certain exemplary embodiments, statistical analysis may be used for variation calling and/or for assembly of a contiguous sequence that is largely based on a known reference, but which comprise alterations or variations from the sequence of the reference, such changes including but not limited to deletions, insertions, mutations, polymorphisms, and duplications or rearrangements of one or more bases. Although the exemplary embodiments may be described for use primarily on mated reads comprising variably gapped reads, the exemplary embodiments may also be configured for use with mated reads that have other predicted or defined spatial relationships, e.g., mate reads having non-gapped reads or where adjacent ends of the mated reads are gapless.

BRIEF DESCRIPTION OF THE DRAWINGS

Figure 1 is a block diagram illustrating a system for calling variations in a sample polynucleotide sequence according to one exemplary embodiment.

Figure 2 is a block diagram illustrating an exemplary mated read.

Figure 3 is a block diagram illustrating mapped mated reads, i.e., mated reads that are mapped to the positions in the reference polynucleotide sequence.

Figure 4A is a diagram illustrating a process for calling variations in mapped mated reads obtained from a sample polynucleotide sequence compared to a reference polynucleotide sequence in accordance with an exemplary embodiment.

Figure 4B is a block diagram graphically depicting generation of a set of one or more sequence hypotheses for each local area.

Figure 4C is block diagram graphically depicting optimization of the sequence hypotheses 412 for each of the local areas 300.

Figure 4D is a diagram graphically illustrating variation calling.

Figure 5 is a flow diagram illustrating the details of the process performed by the variation caller for calling variations in a sample polynucleotide sequence in accordance with an exemplary embodiment.

Figure 6 is a flow diagram illustrating a process performed during computing reference scores using the Bayesian Formulation.

Figures 7A and 7B are diagrams illustrating the process for computing the local de novo intervals using the partial de Bruijn graph.

Figure 8 is a flow diagram illustrating a process for finding optimization intervals according to one exemplary embodiment.

Figures 9A and 9B are flow diagrams illustrating a process for performing optimization using a Bayesian formulation and a de Bruijn graph.

DETAILED DESCRIPTION OF THE INVENTION

The practice of the techniques described herein may employ, unless otherwise indicated, conventional techniques and descriptions of organic chemistry, polymer technology, molecular biology (including recombinant techniques), cell biology, biochemistry, and sequencing technology, which are within the skill of those who practice in the art. Such conventional techniques include polymer array synthesis, hybridization and ligation of polynucleotides, and detection of hybridization using a label. Specific illustrations of suitable techniques can be had by reference to the examples herein. However, other equivalent conventional procedures can, of course, also be used. Such conventional techniques and descriptions can be found in standard laboratory manuals such as Green, et al., Eds. (1999), Genome Analysis: A Laboratory Manual Series (Vols. I-IV); Weiner, Gabriel, Stephens, Eds. (2007), Genetic Variation: A Laboratory Manual; Dieffenbach, Dveksler, Eds. (2003), PCR Primer: A Laborafory Manual; Bowtell and Sambrook (2003), DNA Microarrays: A Molecular Cloning Manual; Mount (2004), Bioinformafics: Sequence and Genome Analysis; Sambrook and Russell (2006), Condensed Protocols from Molecular Cloning: A Laboratory Manual; and Sambrook and Russell (2002), Molecular Cloning: A Laboratory Manual (all from Cold Spring Harbor Laboratory Press); Stryer, L. (1995) Biochemistry (4th Ed.) W.H. Freeman, New York N.Y.; Gait, "Oligonucleoride Synthesis: A Practical Approach" 1984, IRL Press, London; Nelson and Cox (2000), Lehninger, Principles of Biochemistry 3rd Ed., W. H. Freeman Pub., New York, N.Y.; and Berg et al. (2002) Biochemistry, 5th Ed., W.H. Freeman Pub., New York, N.Y.

Note that as used herein and in the appended claims, the singular forms "a," "an," and "the" include plural referents unless the context clearly dictates otherwise. Thus, for example, reference to "a mated read' refers to one or more polynucleotide mated reads, and reference to "probability analysis" includes reference to equivalent steps and methods known to those skilled in the art, and so forth.

Unless defined otherwise, all technical and scientific terms used herein have the same meaning as commonly understood by one of ordinary skill in the art to which the embodiments belongs. All publications mentioned herein are incorporated by reference for the purpose of describing and disclosing devices, formulations and methodologies that may be used in connection with the presently described embodiments.

Where a range of values is provided, it is understood that each intervening value, between the upper and lower limit of that range and any other stated or intervening value in that stated range is encompassed within the embodiments. The upper and lower limits of these smaller ranges may independently be included in the smaller ranges, and are also encompassed within the embodiment, subject to any specifically excluded limit in the stated range. Constant values are used for illustration only and systems for implementing the exemplary embodiments can be configured to work with other values.

DEFINITIONS

The following definitions may be helpful in providing background for an understanding of the invention.

"Polynucleotidd', "nucleic acid', "oligonucleotide", "oligo" or grammatical equivalents used herein refers generally to at least two nucleotides covalently linked together in a linear fashion. A nucleic acid generally will contain phosphodiester bonds, although in some cases nucleic acid analogs may be included that have alternative backbones such as phosphoramidite, phosphorodithioate, or methylphophoroamidite linkages; or peptide nucleic acid backbones and linkages. Other analog nucleic acids include those with bicyclic structures including locked nucleic acids, positive backbones, non-ionic backbones and non-ribose backbones.

The term "reference polynucleotide sequence", or simply "reference", refers to a known sequence of nucleotides of a reference organism. The reference may be an entire genome sequence of a reference organism, a portion of a reference genome, a consensus sequence of many reference organisms, a compilation sequence based on different components of different organisms, a collection of genome sequences drawn from a population of organisms, or any other appropriate sequence. The reference may also include information regarding variations of the reference known to be found in a population of organisms. The reference organism may also be specific to the sample being sequenced, possibly a related individual or the same individual, separately drawn (possibly normal to complement cancer sequence).

'Sample polynucleotide sequence' refers to a nucleic acid sequence of a sample or target organism derived from a gene, a regulatory element, genomic DNA, cDNA, RNAs including mRNAs, rRNAs, siRNAs, miRNAs and the like and fragments thereof. A sample polynucleotide sequence may be a nucleic acid from a sample, or a secondary nucleic acid such as a product of an amplification reaction. For a sample polynucleotide sequence or a polynucleotide fragment to be "derived' from a sample polynucleotide (or any polynucleotide) can mean that the sample sequence/polynucleotide fragment is formed by physically, chemically, and/or enzymatically fragmenting a sample polynucleotide (or any other polynucleotide). To be "derived'from a polynucleotide may also mean that the fragment is the result of a replication or amplification of a particular subset of the nucleotide sequence of the source polynucleotide.

A "Mated read' refers generally to a set of individual nucleotide reads originating from two distinct regions of genomic sequence (arms) located at a distance of a few hundred or thousand bases. The mated read may be generated during sequencing from a fragment of a larger contiguous polynucleotide (e.g., DNA) obtained from the sample organism to be variation called and/or reassembled.

"Mapping" refers to a process which relates a mated read to zero, one or more locations in the reference to which the mate read is similar, e.g., by matching the instantiated mated read to one or more keys within an index corresponding to a location within a reference.

'Bayes' Theorem" refers to a mathematical formula used for calculating conditional probabilities, where probability of a hypothesis H conditional on a given body of data $E$ is the ratio of the unconditional probability of the conjunction of the hypothesis with the data to the unconditional probability of the data alone. The probability of H conditional on $E$ is defined as $P_E(H) = P(H \& E)/P(E)$.

'De Bruijn graph" refers to a graph whose vertices or nodes are sequences of symbols from some alphabet and whose edges indicate the sequences which might overlap.

"Partial de Bruijn graph process" refers to a process that does not compute an entire De Bruijn graph, but basically follows an initial graph of vertices (preferably derived from the reference) and determines if there are branches. Any branching beyond a certain threshold may be taken to indicate a possibility of a variation being present in a local area.

"reassemble" and "resequence" refer to methods of assembling mapped mated reads against the reference to build a sequence that is similar, but not necessarily identical, to the original reference. This is in contrast to de novo assembly in which mated reads are assembled together to form a new previously unknown sequence.

In the following description, numerous specific details are set forth to provide a more thorough understanding of the present embodiments. However, it will be apparent to one of skill in the art that the present embodiments may be practiced without one or more of these specific details. In other instances, well-known features and procedures well known to those skilled in the art have not been described in order to avoid obscuring the embodiments.

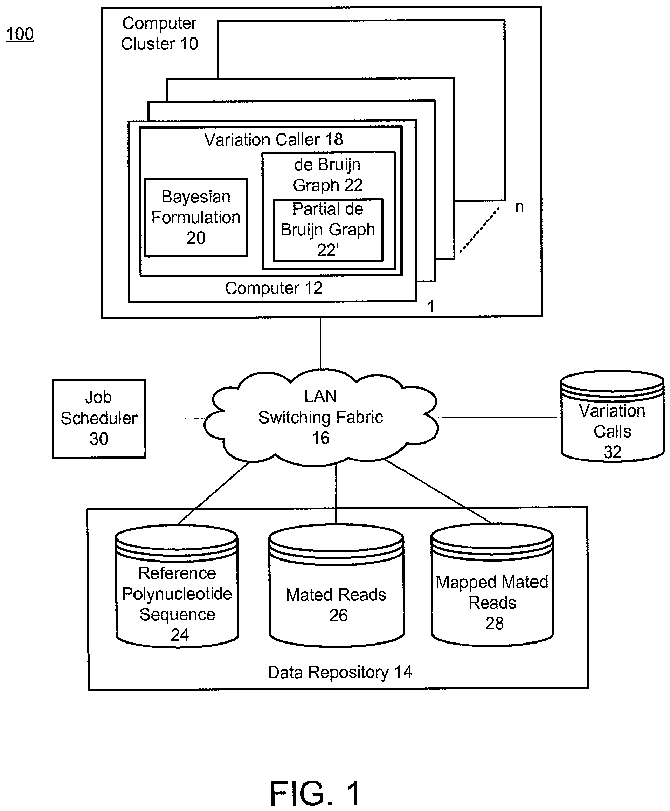

Figure 1 is a block diagram illustrating a system for calling variations in a sample polynucleotide sequence according to one exemplary embodiment. In this embodiment, the system 100 may include a computer cluster 10 of 1-n computers 12 and a data repository 14. For example, in one particular embodiment, the system may include 32 computers. The computers 12 may be connected to the data repository 14 via a high-speed local area network (LAN) switching fabric 16. At least a portion of the computers 12 may execute instances of a variation caller 18 in parallel. The variation caller 18 may include a Bayesian formulation 20 component and a de Bruijn graph 22 component.

The data repository 14 may store several databases including one or more databases that store a reference polynucleotide sequence 24, mated reads 28 obtained from a sample polynucleotide sequence during biochemical processes, and mapped mated reads 28 that are generated from the mated reads 28.

The reference polynucleotide sequence 24 (hereinafter referred to as simply the reference) refers to a known sequence of nucleotides of a reference organism (e.g., a known genome). This includes references comprising sequences having known variations at one or more location within the genome. A polynucleotide molecule is an organic polymer molecule composed of nucleotide monomers covalently bonded in a chain. Deoxyribonucleic acid (DNA) and ribonucleic acid (RNA) are examples of polynucleotides with distinct biological function. The genome of an organism is the entirety of an organism's hereditary information, which is encoded as DNA or RNA. A haploid genome contains one copy of each hereditary unit of the organism. In diploid animals such as mammals, the genome is a series of complementary polynucleotides comprising two copies of the majority of the hereditary information organized as sets of chromosomes having discrete genetic units, or alleles. Each copy of the allele is provided at a specific position on an individual chromosome, and the genotype for each allele in a genome comprises the pair of alleles present at particular positions on homologous chromosomes that determine a specific characteristic or trait. Where a genome comprises two identical copies of an allele it is homozygous for that allele, and when the genome comprises two different alleles it is heterozygous for that locus. The DNA itself is organized as two strands of complementary polynucleotides. The strand that corresponds to the reference may be referred to as "positive", and the strand complementary to the reference may be referred to as "negative" in the following discussion.

The reference 24 may be an entire genome sequence, a portion of a reference genome, a consensus sequence of many reference organisms, a compilation sequence based on different components of different organisms, or any other appropriate sequence. The reference 24 may also include information regarding variations of the reference known to be found in a population of organisms.

The mated reads 26 may be obtained during a sequencing process performed on polynucleotide sequence of a sample organism, e.g., a nucleic acid sequence from a gene, genomic DNA, RNA, or a fragment thereof, that is to be analyzed. The mated reads 26 can be obtained from a sample comprising an entire genome, such as an entire mammalian genome, more specifically an entire human genome. In another embodiment, the mated reads 26 may be specific fragments from a complete genome. In one embodiment, the mated reads 26 may be obtained by performing sequencing on amplified nucleic acid constructs, such as amplimers created using polymerase chain reaction (PCR) or rolling circle replication. Examples of amplimers that may be used are described, for example, in U.S. Pat. Publication Nos. 20090111705, 20090111706 and 20090075343.

The mapped mated reads 28 refer to the mated reads 26 that have been mapped to locations in the reference 24, as explained further below in the Mapping section.

In one embodiment, the variation caller 18 comprises program instructions that perform statistical analysis, such as probability analysis or other methods permitting scoring of alternative hypotheses, on the reference 24 and on the mapped mated reads 28 based in part on a combination of evidential reasoning performed by the Bayesian formulation 20, and de Bruijn graph based algorithms performed by both the de Bruijn graph 22 component and a partial de Bruijn graph 22' component.

Using the statistical analysis, the variation caller 18 generates and scores sequence hypotheses for the purpose of identifying and calling variations or differences detected in a sequence of the mapped mated reads 28 in relation to the reference 24. In one embodiment, the variation caller 18 is capable of identifying and calling large-scale structural variations comprising sequences of deletions, insertions, mutations, polymorphisms, and duplications or rearrangements of one or more bases, and the like.

The variation caller 18 may output a variation calls 32 file, list or other data structure containing the identified variations, each describing a manner in which parts of the sequence of mapped mated reads 28 are observed to differ from the reference 24 at or near specific locations. In one embodiment, the variations caller 18 may also output a list of no-called intervals that could not be called due to computational uncertainties.

In a further embodiment, the variation caller 18 may be configured to use the probability analysis or other approach to scoring alternative hypotheses to reassemble or resequence the polynucleotide sequence of the sample organism from the mapped mated reads 26, where the assembled polynucleotide sequence is based substantially on the reference 24, but includes the identified variations.

In one embodiment, the variation caller 18 may be implemented as a single executable that links to libraries (not shown) to perform specific tasks. In another embodiment, the variation caller 18 may be implemented as separate application modules that communicate with each other. In addition, the functionality of the variation caller 18 may be distributed across a greater or lesser number of software modules/components than that shown in Figure 1.

To speed processing, the computer cluster 10 may be configured such that instances of the variation caller 18 executing on different computers 12 operate on different portions of the reference 24 and the mapped mated reads 26 in parallel. The job scheduler 30 is responsible for assignment of jobs or packets of work across the various computers 12 in the computer cluster 10.

The computers 12 may include typical hardware components (not shown) including, one or more processors, input devices (e.g., keyboard, pointing device, etc.), and output devices (e.g., a display device and the like). The computers 12 may include computer-readable/writable media, e.g., memory and storage devices (e.g., flash memory, a hard drive, an optical disk drive, a magnetic disk drive, and the like) containing computer instructions that implement the functionality disclosed when executed by the processor. The computers 12 may further include computer writeable media for implementing the data repository 14 and for storing the variation calls 32. The computers 12 may further include wired or wireless network communication interfaces for communication. In one embodiment, the computer cluster 10 may be implemented as a commodity Intel/Linux cluster that scales horizontally with additional processors.

DATA GENERATION

In some implementations, biochemical techniques may be used to generate the mated reads 26 obtained from the sample polynucleotides of an organism to be analyzed. In one embodiment, the biochemical techniques provide the data in discrete but related sets, such that contents of the mated reads 26 may include predicted spatial relationships and/or separation variations. The relationships may be determined based on existing knowledge about the biochemical process used to generate the mated reads 26 (i.e., based on sequences that would be expected to be obtained if the biochemical process were applied to a sample), empirical estimates based on preliminary analysis of the sequence data of the mated reads 26 or subsets thereof, estimation by experts, or other appropriate techniques.

Numerous biochemical processes can be used to generate the mated reads 26 for use with the present assembly methods. These include, but are not limited to hybridization methods as disclosed in US Pat. Nos. 6,864,052; 6,309,824; 6,401,267; sequencing-by-synthesis methods as disclosed in US Pat. Nos. 6,210,891; 6,828,100, 6,833,246; 6,911,345; 7,329,496 and Margulies, et al. (2005), Nature 437:376-380 and Ronaghi, et al. (1996), Anal. Biochem. 242:84-89; ligation-based methods as disclosed in US Pat. No. 6,306,597, WO2006073504, WO2007120208, nanopore sequencing technology as disclosed in US Pat. Nos. 5,795,782, 6,015,714, 6,627,067, 7,238,485 and 7,258,838 and U.S. Pat Appln Nos. 2006003171 and 20090029477, and nanochannel sequencing technology as disclosed in US Pat. Appln No 20090111115. In a specific implementation" a Combinatorial Probe Anchor Ligation (cPAL) process is used in some embodiments (see US Pat App Publication Nos. 20080234136 and 20070099208).

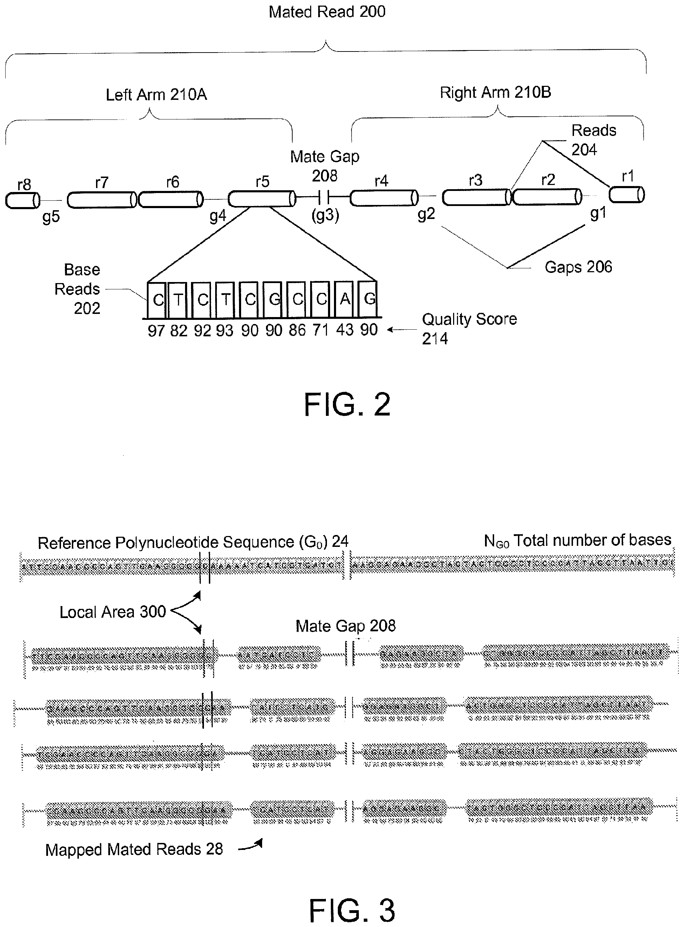

Figure 2 is a block diagram illustrating an exemplary mated read 200. In one embodiment, the mated read 200 is generated during sequencing from a fragment of a larger contiguous polynucleotide (e.g., DNA) obtained from the sample organism to be variation called and/or reassembled. Only a portion of the bases of the original fragment are read in each mated read 200. Bases from the fragment are referred to as base reads 202 after the biochemical operation that generates the mated read 200. These base reads 202 from a fragment thus constitute a small fraction of the bases in the original larger polynucleotide.

A single sequencing experiment may generate $N_D$ mated reads 26 from the sample polynucleotide sequence (e.g., a sample genome G), and each mated read 200 may only include $n_D$ bases, referred to as base reads 202, of the original fragment. This results in a total of $n_D N_D$ base reads. Assuming there are the $N_G$ bases of the sample genome G, the average coverage per allele is c = $n_D N_D / N_G$. This means that, neglecting coverage bias, each of the $N_G$ bases of the sample genome G (counting both alleles in diploid areas) receives an average of c base reads. In specific sequencing experiments, $c \approx 25$ and the number of bases read of the original fragment, $n_D = 70$, and therefore the number of mated reads 200, $N_D = 2 \times 10^9$.

In its simplest form, each individual base read 202 consists of one of four symbol values A, C, G, T(or U), corresponding to the 4 bases found in the nucleotides of DNA or RNA, respectively. The base values for some the base reads 202 can occasionally be missing, in which case the base values are represented by the symbol N and referred to as no-calls. In one embodiment, each base read 202 is associated with a quality score 214 (a number between 0 and 1; or 0 and 99, for example) that indicates the estimated quality of that base read 202. The variation caller 18 may use the quality score to determine a contribution of evidence from individual base reads 202 within a read 204.

In one embodiment, the relative positions of the base reads 202 being read during the biochemical processes are known only approximately and are in the form of $m_d$ contiguous reads 204, numbered from 0 to $m_d$ - 1 The biochemical processes used to generate the mated reads 26 may result in reads 204 of various lengths, including different lengths within a single mated read 200. The $i^{th}$ read 204 has a length of $l_i$ base reads 202, and $l_i$ in one embodiment, may be the same for all related mated reads 200 in a given sequencing project. This restriction can be optionally relaxed at the cost of some additional notational complexity.

In one embodiment, the mated read 200 comprises multiple gaps in the data. For example, the $m_d$ reads 204 may be separated by $m_d$ -1 gaps 206 (g1 to g5), numbered from 0 to $m_d$ - 2. The value of each of the gaps 206 for a specific mated read 200 may be variable and unknown. However a statistical distribution of gap values is available *a priori* or from empirical sampling by mapping.

It should be noted that the mated read 200 shown in Figure 2 is for illustrative purposes only and different library (i.e., read) architectures can have varied number of reads 204 and correspondingly, intra-read gaps between the reads can be of variable lengths, and gaps can occur between any of the reads.

In one embodiment, the mated read 200 may include a mate pair gap 208 that divides the mated read 200 into two subset elements referred to as a left arm 210A and a right arm 210B (collectively referred to as arms 210). The mate pair gap 208 may encompass a relatively large number of values, such as for example, a distribution peaked at approximately 400-500 bases and a width of 100 bases. In one embodiment, the left arm 210A and the right arm 210B may comprise $m_{d,l}$ 204, respectively.

In a very specific embodiment, the biochemical processes produce left and right arms 210 having 35 base reads 202 each, and in this embodiment, the left and right arms 210 may each include 4 contiguous reads 204 ($m_{d,1}$ = $m_{d,2}$ = 4). The 4 contiguous reads 204, in turn, each comprise three 10-base reads and one 5-base read. The 5-base read in the mated read 200 may be likely the outermost one relative to the mate pair gap 108 (e.g., r1 and r8).

The intra-arm gaps 206 can each only take a small number of values, which are small positive or negative integers. Negative gap values correspond to the same genomic base being read more than once within a single mated read 200. In another embodiment, more than two arms may be mated, which might be appropriate if there are additional gaps that can adopt a large range of values.

In one embodiment, the $m_d$ - 1 gap values $g$ of a particular mated read 200 can be partitioned into three groups - $g_l$, $g_m$, and $g_r$ of $m_{d,1}$-1, 1, and $m_{d,2}$ - 1 values respectively, corresponding to the left arm 210A, the mate pair gap, and the right arm 210B. The probability distributions for the three groups of gap values are assumed to be uncorrelated, and therefore the probability distribution for the gap values $g$ can be written as a product:

$$P(g) = P(g_1)P(g_m)P(g_2).$$

Although the distributions of the entire mated read 200 can be written as a product, the first and third gap value probability distributions $P(g_1)$ and $P(g_2)$ do not necessarily decouple into products of probabilities for the individual gap values. In addition, this is based on an assumption that the gap probabilities are independent of other nearby sequences. It will be apparent to one skilled in the art upon reading the present disclosure that removing this assumption can be done with some additional notational complexity.

GENOME MODEL

In resequencing assembly of an entire genome, it is assumed that the sample being sequenced, e.g., genome $G$, is very similar to the known reference 24, e.g., genome $G_0$. The reference genome $G_0$ can be provided as a set of contigs (from *contiguous*), which is a set of non-overlapping polynucleotide segments derived from a genetic source. In more complex genomes such as mammalian genomes, these contigs may correspond to subsets of chromosomes. However, since all contigs are much longer than the length of each of the fragments from which the mated reads 26 originate, for the purpose of this discussion $G_0$ will be treated as if $G_0$ comprises a single contiguous sequence of bases. $G_{0,i}$ or $G_0(i)$ shall be used to indicate a base at position $i$ in $G_0$.

Each section of $G_0$ has an associated ploidy $p$, which is the number of alleles of a particular genetic unit present in the genome. The ploidy is 2 in most regions of the genomes of diploid organisms, including human genomes. Greater or lesser ploidy values are also possible in normal genomes; for example, most of the "Y" chromosome in humans is haploid, and abnormal genomes, including genomes from cancerous cells or from individuals having genetic abnormalities often have regions with unusual ploidy. The p alleles of a genome section are not necessarily equal to each other, but are all very similar to the corresponding section of $G_0$. In specific aspects, the ploidy of each region is treated for analysis's sake as if it were known in advance, although such an assumption is not strictly necessary for the methods of the described embodiments.

The term $N_{G_0}$ is used to indicate the total number of bases in the genome, summed over all alleles, that is used for assembly of the resequencing data. For the normal, diploid human genome this number is approximately $6 \times 10^9$.

MATED READ PROBABILITY MODEL

For the purposes of statistical calculations used in variation calling and raassembly, the mated read 200 generation process is modeled as follows.

Each mated read 200 originates at a random position on one of the two alleles of a sample genome G being sequenced, on one of the two complementary strands. Due to coverage bias, the originating genomic position, strand, and allele are not equally probable. The probability of a mated read 200 having its first base at a given position $x$ of strand $s$ of allele $a$ is taken to be

$$P(x,s,a) = \frac{1}{2N_G} b(x,s,a),$$

where $b(x, s, a)$ is an unknown coverage bias function with mean 1. In most of the description below and in the variation calling process an assumption is made that $b(x, s, a) = 1$, and coverage bias is not taken into consideration. In this case, $P(x, s, a)$ is constant, and $P(x, s, a) = 1/(2N_G)$, and is equal to the inverse of the total number of bases in the two strands of the DNA of the genome.

BASE READS

The quality score 214 of a base read 202 can be converted into an estimated error rate , $\epsilon_i$, e.g., using statistics obtained during mapping of the mated read 200 or elements thereof to the reference 24, explained below, or estimates derived directly from image analysis. As the simplest possible assumption, a base from the reference 24 (e.g., a genomic base) equals a base read 202 in a mated read 200 with probability 1- $\epsilon$, and any of the three remaining possibilities with probability $\epsilon/3$. This is based in part on the implicit assumption that individual base reads 202 are uncorrelated from each other. As is apparent from the methods of this disclosure, in another embodiment this assumption can be lifted in favor of a different error model.

More formally, if $b_g$ is a true genomic base being read and $b_d$ is a base as read in the mated read 200, the probability of the latter conditional to the former is a $4 \times 4$ error matrix given by

$$P\big(b_d\big|b_g\big) = (1 - \epsilon)\delta\big(b_g, b_d\big) + \big[1 - \delta\big(b_g, b_d\big)\big]\epsilon/3$$

where the Kronecker $\delta$ symbol is 1 if its arguments are identical and 0 otherwise.

More complex error models are possible, in which each base read comes with 4 "weight" which are estimates of $P(b_g|b_d)$. The Bayesian formulation further below may be extended to allow for such estimates. In another embodiment, error probabilities can be modeled conditional on flanking sequence, or error probabilities conditional on whether other bases are in error, etc.

MAPPING

The mapped mated reads 28 may be created from the data set of the mated reads 26. High-speed mapping software, e.g., a map-to-reference component (not shown), may be used to map the raw mated reads 26 to base positions in the reference 24 where the mated reads 26 are likely to occur. The map-to-reference component may output the mapped mated reads 28 in the form of locations of the mated reads 26 in the reference 24. The mapping may be tolerant of small variations from the reference 24, such as those caused by individual genomic variation, read errors or unknown bases in reads 202.

Figure 3 is a block diagram illustrating mapped mated reads 28. Mated reads are mated reads that have been mapped to the positions in the reference 24. In one embodiment, a mapped mated read 28 may comprise a mated read that has only one arm 210 aligned to the reference, i.e., half mapping. More specifically, a mated read is considered to map to a position and strand in the reference 24 when the reads 204 of an arm 210 are sufficiently similar to the sequence of the reference 24 starting at that position and strand for the similarity to be recognized by a mapping process such as described in the patent applications listed in the following paragraph. From such a mapping it may be inferred that the mated read may have been derived from the corresponding portion of the sample of interest. In another embodiment, a mapped mated read 28 may comprise a mate read that has both arms 210 aligned to a specified location on the reference, i.e., full mapping. To support assembly of larger variations, including large-scale structural changes or regions of dense variation, the left and right arms 210 of the mated reads 26 may be mapped separately, with mate pairing constraints evaluated after alignment.

In one embodiment, the map-to-reference component may perform the mapping process as disclosed in the following provisional patent applications: "OLIGOMER SEQUENCES MAPPING', Application Number 61/149,670, filed February 3, 2009 (CGI002PRV); "INDEXING A REFERENCE SEQUENCE FOR OLIGOMER SEQUENCE MAPPING', Application Number 61/149,665, filed February 3, 2009 (CGI003PRV); and "OLIGOMER SEQUENCES MAPPING', Application Number 61/149,689, filed February 3, 2009 (CGI004PRV), all assigned to the assignee of the present application.

Summarizing the process, mate-paired arm reads may be aligned to the reference 24 using a two-stage process. First, the left and right arms 210 may be aligned independently using indexing of the reference genome. In one embodiment, this initial search may find all locations in the reference 24 that match the left arm 210A with at most two single-base substitutions, but may find some locations that have up to five mismatches. Second, for every location of a left arm identified in the first stage, the right arm 210B is subjected to a local alignment process, which may be constrained to a genomic interval informed by the distribution of the mate distance (e.g., 0 to 700 bases away). The same local alignment process may then be repeated for arm 210A using the locations of arm 210B. At both stages, the alignment of a gapped arm read 210 may be performed by trying multiple combinations of gap values. If a mated read 200 has any consistent locations of arms 210 (that is, left and right arms are on the same strand, in the proper order and within an expected mate-distance distribution), then only these locations may be retained. Otherwise, all locations of the arms 210 may be retained. In either case, for performance reasons, the number of reported locations for every arm 210 may be limited, e.g., to 50 locations in one embodiment.

In one embodiment, storage records for the mapped mated reads 28 may include a list of arm mappings to the reference polynucleotide sequence ($G_0$) 24 sorted in reference order to identify sequences that can possibly contribute to the Bayesian computation of $L(G)$, explained further below. The records may contain not only mapping information, but also the base reads 204 and quality score 214 of the corresponding mated read 200.

VARIATION CALLING PROCESS

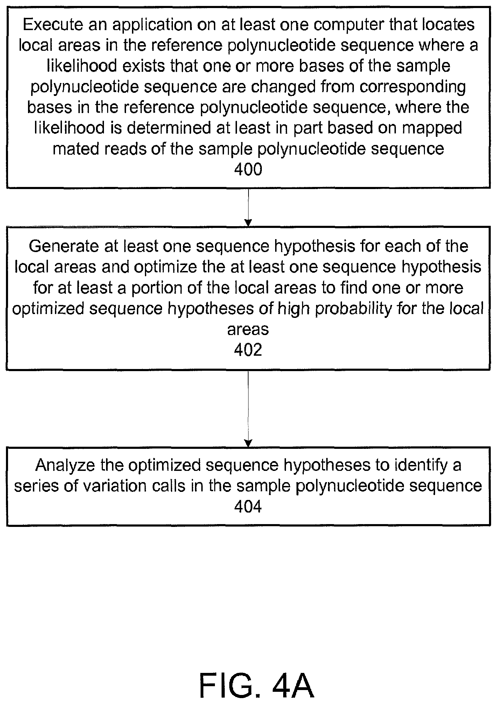

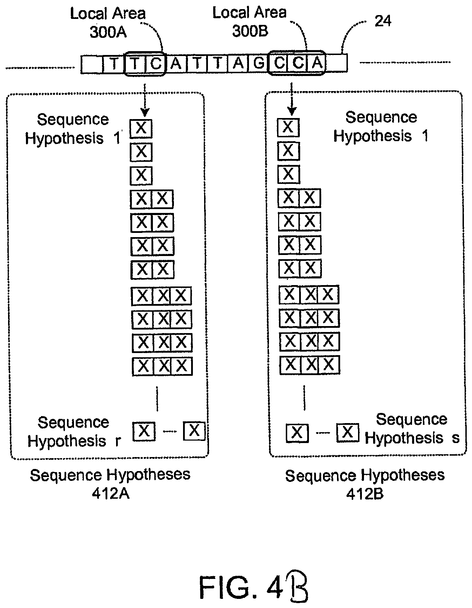

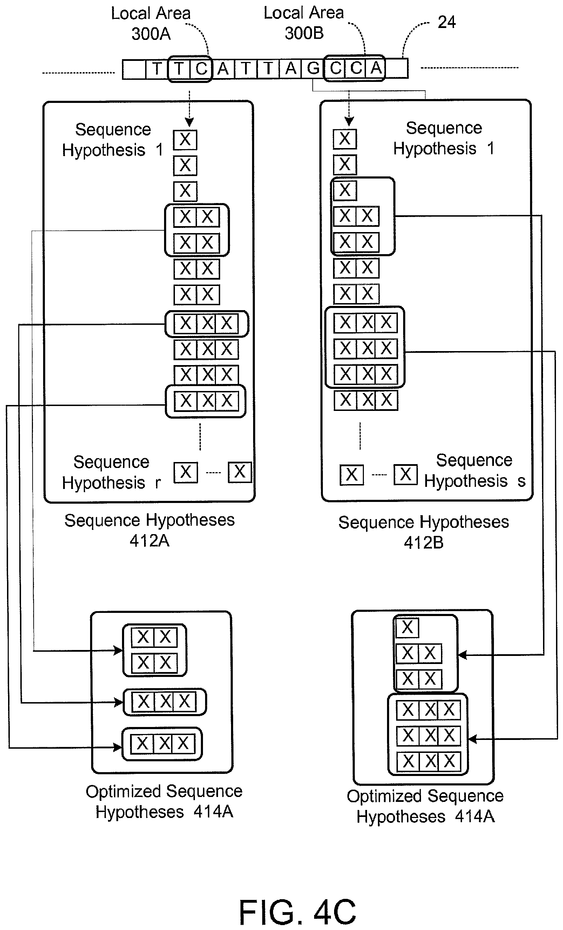

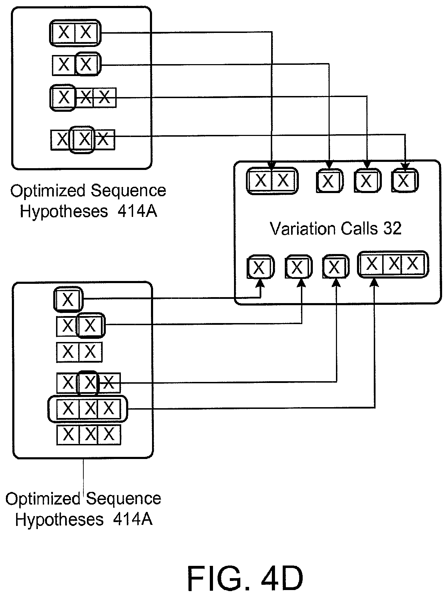

Figure 4 is a diagram illustrating a process for calling variations in a sample polynucleotide sequence with respect to a reference polynucleotide sequence 24 in accordance with an exemplary embodiment. The process may include executing an application on at least one computer 12 that locates local areas in the reference polynucleotide sequence where a likelihood exists that one or more bases of the sample polynucleotide sequence are changed from corresponding bases in the reference polynucleotide sequence, where the likelihood is determined at least in part based on mapped mated reads of the sample polynucleotide sequence (block 400).

An example of a local area 300 is shown Figure 3 by vertical bars spanning across corresponding locations in the reference 24 and the mated reads 28. This local area 300 identifies a location where the mapped mated reads 28 are likely to have variations or changes in one or more bases from the corresponding bases in the reference polynucleotide sequence 24. The local area 300 may comprise one or a sequence of base positions.

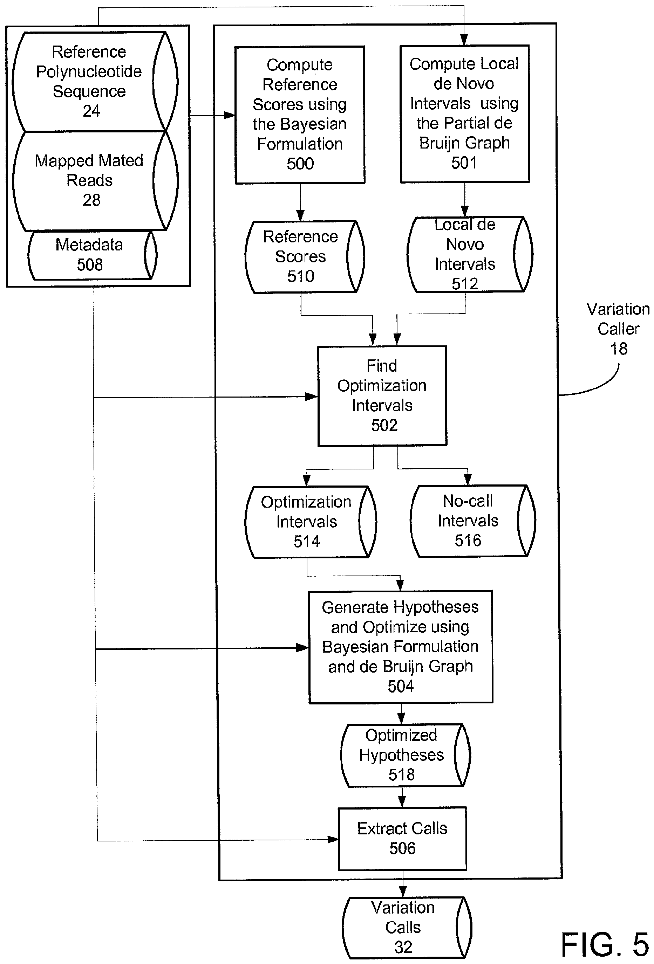

In one embodiment, locating the local areas 300 likely to have changes from the reference is performed by the variation caller 18 using at least in part using the Bayesian formulation 20 component during a compute reference scores procedure and using the partial de Bruijn graph 22 component to compute local de novo intervals, as further described below with reference to Figure 5. In one embodiment, small or individual local areas 300 of likely change may be combined to form larger local areas called optimization intervals during a find optimization intervals procedure, as described in Figure 5.

The variation caller 18 generates at least one sequence hypothesis for each of the local areas 300 and optimizes the at least one sequence hypothesis for at least a portion of the local areas to find one or more optimized sequence hypotheses of high probability for the local areas (block 402). In one embodiment, generating the sequence hypothesis for a local area may include traversing each base position in the local area and iteratively changing the base with every possible alternative base value, including an insertion or deletion at that position, which may result in multiple sequence hypotheses being generated for each local area 300.

Figure 4B is a block diagram graphically depicting generation of a set of one or more sequence hypotheses for each local area. In this example, two local areas of likely change 300A and 300B (collectively referred to as local areas 300) have been identified in the reference polynucleotide sequence 24. For each of the local areas 300A and 300B, a set of sequence hypotheses 412A and 412B, respectively (collectively referred to as sequence hypotheses 412), are generated by changing base values in the respective local area 300.

Figure 4C is block diagram graphically depicting optimization of the sequence hypotheses 412 for each of the local areas 300. In one embodiment, optimizing the sequence hypotheses 412 may include computing a probability ratio for each individual sequence hypothesis therein. The sequence hypotheses 412 resulting in a high probability ratio for each of the local areas 300 may be used to form a set of optimized sequence hypotheses 414A and 414B (collectively referred to as optimized sequence hypotheses 414). In one embodiment, the sequence hypothesis from the optimized sequence hypotheses 414A that maximize the probability ratio is applied to the local area 300A. According to one embodiment, the optimization is performed during a sequence hypothesis generation and optimization procedure that utilizes the Bayesian formulation 20 component and the de Bruijn graph component 22, as explained further with reference to Figure 5.

The variation caller 18 analyzes each set of the optimized sequence hypotheses to identify a series of variation calls in the sample polynucleotide sequence, where the variation calls are strongly supported by the mapped mated reads (404). In one embodiment, a series of variation calls may be identified during an extract calls process that may comprise indicating variations detected in the bases of the mapped mated reads 28 of the sample polynucleotide sequences 26 in relation to the reference 24 at or near specific locations. In one specific embodiment, the variation calls may be identified based at least in part on statistical probability analysis.

Figure 4D is a diagram graphically illustrating variation calling. In this example, all or a subset of the optimized sequence hypotheses 414 are individually examined and variation calls 32 are made as to which part or parts of the optimized hypotheses 414 most correctly describes the corresponding sequence in the reference 24. In one embodiment, the variation calls are made by an extract calls component, as further explained in Figure 5.

Figure 5 is a flow diagram illustrating the details of the process performed by the variation caller 18 for calling variations in a sample polynucleotide sequence in accordance with an exemplary embodiment. The variation caller 18 may include five primary processes: Compute Reference Scores Using the Bayesian Formulation 500, Compute Local De Novo Intervals Using the Partial De Bruijn Graph 501, Find Optimization Intervals 502, Generate Hypotheses and Optimize using the Bayesian Formulation and the De Bruijn Graph 504, and Extract Calls 506. Using these processes, the variation caller 18 may obtain a reassembled sample polynucleotide sequence, e.g., genome ($G$) by iteratively maximizing the genome's a posteriori probability $P(G/MtdRds)$, which accounts for all the mapped mated reads (MtdRds) 28.

The process may begin by the variation caller 18 receiving the reference polynucleotide sequence ($G_0$) 24 and the mapped mated reads 28 as read-only inputs to all five processes. The variation caller 18 may also receive as a read-only input metadata 508, which may describe the job, i.e., identifies the reference 24, a location of the input files, defines a structure of the mapped mated reads 28, and the like. As stated previously, the reference 24 comprises a known sequence of nucleotides of a reference organism, e.g., National Center for Biotechnology Information (NCBI) Build 36 reference human genome. The mapped mated reads 28 each comprise multiple contiguous reads 204 separated by variable gaps. Each of the reads 204 constituting a mapped mated read 28 includes multiple bases located at respective base positions in the mapped mated read. These bases are also mapped to base positions in the reference 24.

Using the inputs, the Compute Reference Scores Using the Bayesian Formulation 500 process computes reference scores 510 and the Compute Local De Novo Intervals Using the Partial De Bruijn Graph 501 process computes local de novo intervals 512.

REFERENCE SCORES 510

In one embodiment, the Compute Reference Scores Using the Bayesian Formulation 500 (hereinafter Compute Reference Scores 500) process aide in identifying the local areas 300 of likely change. The process computes for each base of the reference 24, probability ratios between all possible 1-base variations at that base position and the reference base at that base position. In one embodiment, each 1-base variation may be referred to as an initial hypothesis.

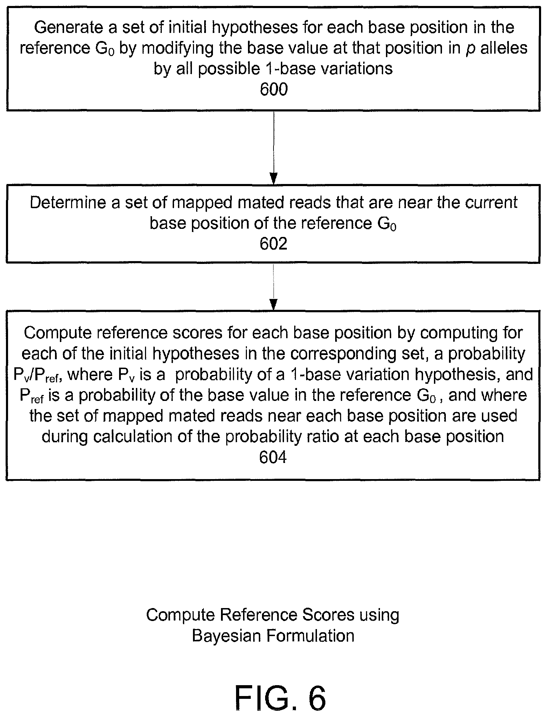

Figure 6 is a flow diagram illustrating the process performed during computing reference scores using the Bayesian Formulation. The Compute Reference Scores 500 process may include generating a set of initial hypotheses for each base position in the reference $G_0$ by modifying the base value at that position in p alleles by all possible 1-base variations (block 600). The modifications may include a single base change (to reflect a potential single nucleotide polymorphism (SNP) in the sequence), and insertions and deletions (collectively termed indels). In addition to single base changes, an initial hypothesis may include indels of up to 10 bases when the modification is made in a genomic region comprising tandem repeats, e.g., three bases may be inserted or deleted in a region of triplet base tandem repeats.

The Compute Reference Scores 500 process determines a set of mapped mated reads that are near (and possibly overlap) the current base position of the reference $G_0$ (block 602). In one embodiment, the mapped mated reads 28 are identified that have one arm mapped to a location approximately the expected length of the mate pair gap away from the current base position in the appropriate direction and strand to satisfy the expected mate pair relationship.

The Bayesian formulation 20 is used to compute reference scores for each base position of the reference $G_0$ by computing for each of the initial hypotheses in the corresponding set, a probability ratio $P_v/P_{ref}$, where $P_v$ is a probability of a 1-base initial hypothesis, and $P_{ref}$ is a probability of the base value in the reference $G_0$, and where the set of mapped mated reads near each base position are used during calculation of the probability ratio at each base position (block 604).

In a further embodiment, assuming the sample polynucleotide sequence comprises a genome $G$, then each of the reference scores may comprise a logarithmic likelihood ratio $L(G)$, where $L(G) = log(P_v/P_{Ref})$. In a further embodiment, the logarithmic likelihood ratio may be expressed (described more fully below) as:

$$L(G) = log\frac{P(G|MtdRds)}{P(G_0|MtdRds)}.$$

In most cases, $L(G)$ is computed to be large and negative, expressing the fact that no variation from $G_0$ is present at that position. However, at positions where a 1-base variation is present, $L(G)$ will be computed to be a positive number. This computation would be sufficient to call and assemble one-base variations, but the situation is complicated by the possibility of longer variations. In local areas 300 where a longer variation is present, $L(G)$ for one-base variations can still be positive, but at a much lower value than in local areas where one-base variations are present. A low value positive number in $L(G)$ can be used to recognize the possibility of the presence of a variation and to identify a local area 300. Positions in the reference 24 where one or more of the probability ratios exceeds a threshold may be output as local areas 300 likely to have changed relative to the reference.

An output of the Compute Reference Scores 500 is a file containing a table of computed reference scores 510. Each row of this table may refer to a position of the reference genome, and contain reference scores for that position, where the reference scores are the computed probabilities of all possible 1-base variation hypotheses, in homozygous or heterozygous form, at that position. In one embodiment, for each of the possible hypothesis, a $L(G) = \log (P_V/P_{Ref})$, may be stored as the reference score.

In one embodiment, each row of the table of reference scores 510 may contain:

Fields to identify the position of the reference genome for that row. This can include a chromosome identification and position, or equivalent information;

Three fields containing $L(G) = \log (P_V/P_{Ref})$ for all three possible homozygous SNPs at the position of interest;

One field containing $L(G) = \log (P_V/P_{Ref})$ for a homozygous deletion at the position of interest;

Four fields containing $L(G) = \log (P_V/P_{Ref})$ for all four possible homozygous one base insertions immediately before the position of interest; and

An additional 8 fields replicating the previous 8 fields, but for heterozygous variations rather than homozygous ones.

In some embodiments, it may be useful to report as the reference score for a given position the score of the initial hypothesis having the highest reference score of any of the evaluated substitutions, including the possibility of scoring multi-base modifications such as tandem repeat copy changes for short tandem repeats or sequences suggested by the local de novo process.

LOCAL DE NOVO INTERVALS 512

According to the exemplary embodiment, the Compute Local De Novo Intervals Using the Partial De Bruijn Graph 501 process (hereinafter Compute Local De Novo Intervals 501) complements the Compute Reference Scores 500 process, constituting another means for locating local areas 300 in the sample polynucleotide sequence likely to have changes. This process may generate variations beyond single base changes by finding mapped mated reads 28 that map to a reference position, but also extend past that position. The Partial De Bruijn Graph 501 process refers to a process that does not compute an entire de Bruijn graph, but basically follows an initial graph of reference vertices and determines if there are branches. Any branching beyond a certain threshold may be taken to indicate a possibility of a variation being present in a local area 300. Such a local area 300 may be subjected to further analysis (e.g., optimization, described below) in order to determine what variations, if any, are present.

As an overview, the local de novo process initializes a partial de Bruijn graph with reference vertices that are created from base sequences (e.g., each 30 bases in length) from the reference 24. The graph is then iteratively augmented. For each vertex already in the graph, a set of mapped mated reads are found that map well to the corresponding reference vertex, but include a base extension extending beyond either end of the reference vertex by any possible 1-base value (A, C, G or T/U). In one embodiment, a mapped mated read can be considered to map well to the corresponding base sequence when at least 14 bases of the read match the sequence and at most one base of the read mismatches the sequence. In one embodiment, the procedure calculates for each of the base extensions an extension strength representing an amount of support for extending the reference vertex by each 1-base value based at least in part on the number of mapped mated reads that have the same extension and the number of matches and mismatches of those mapped mated reads with the sequence of the vertex being processed. Each such 1-base extension corresponds to a possible vertex in the graph. The base extensions having the highest extension strength and that are not yet in graph are added. In one embodiment, the graph building procedure for constructing the partial de Bruijn graph is recursive, but any procedure that correctly repeats the necessary steps can also be used.

In one embodiment of the graph building procedure, extension strength for each of the top base extensions is computed in a depth-first manner in one direction, and a new edge and a new vertex is creating after each computation for the base extensions having extension strength above a threshold. If there are no base extensions having the extension strength above the threshold in a path, a failure is returned for the path. If the computation of the graph building procedure creates a new branch vertex that is equal to the base sequence of one of the reference vertices and that is consistent with a SNP or short indel (approximately 50 bases or shorter), the computation ends and the path is returned.

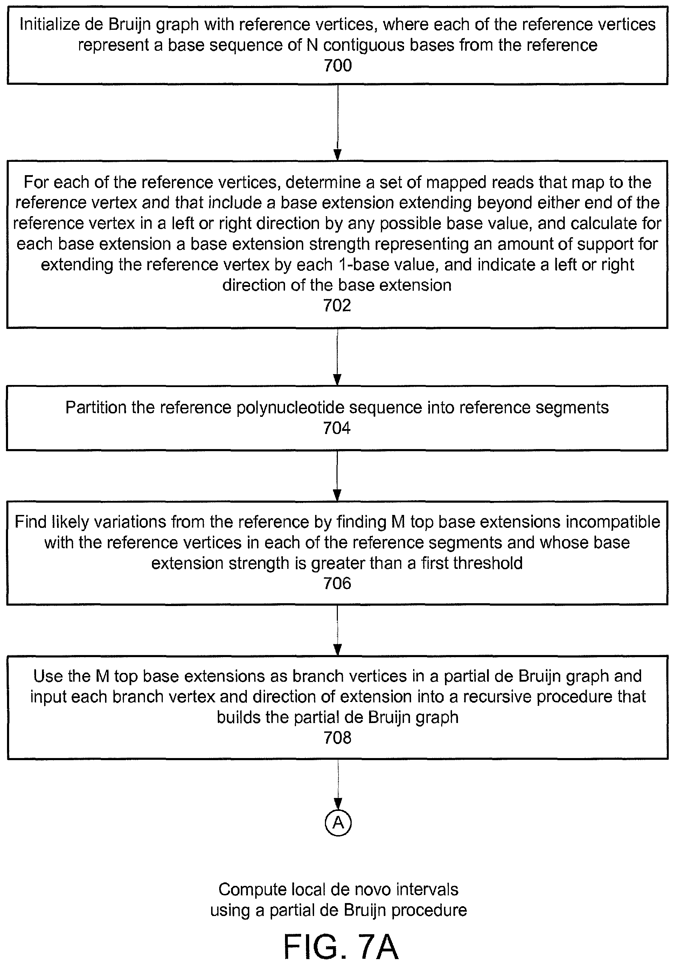

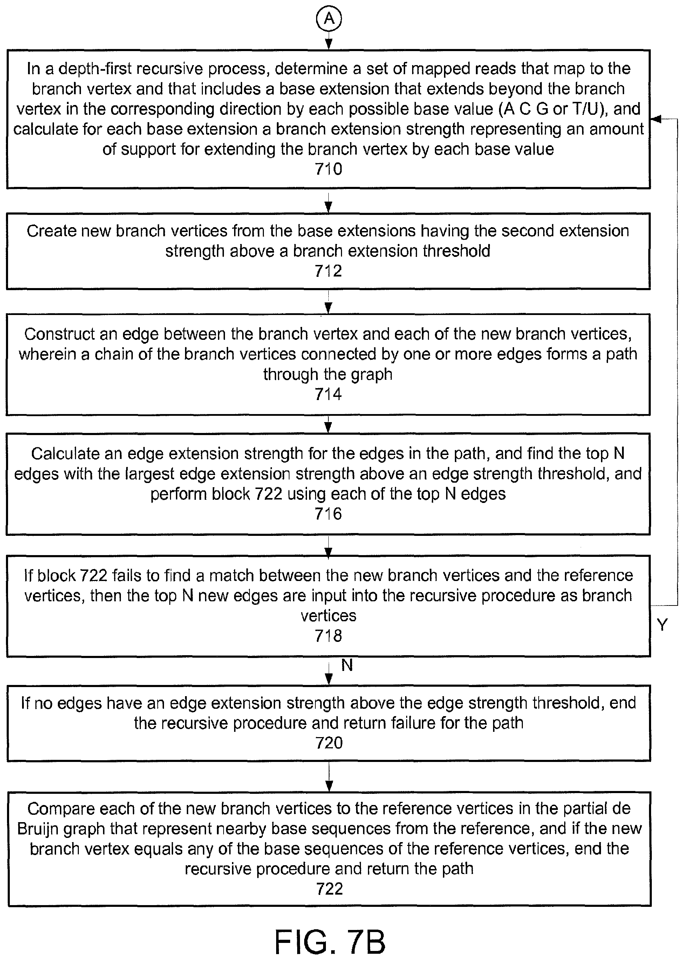

Figures 7A and 7B are diagrams illustrating details of the process for computing local de novo intervals using the partial de Bruijn graph. The process may include initializing a de Bruijn graph with reference vertices, where each reference vertex represents a base sequence of N contiguous bases from the reference genome (block 700). In one embodiment, each base sequence may overlap an immediately adjacent base sequence, e.g., sharing all but 1 base, for example. In one embodiment, the reference vertices may comprise base sequences of 30 bases (N=30), for example.

For each of the reference vertices, a set of mapped reads is determined that map to the reference vertex and that include a base extension extending beyond either end of the reference vertex in a left or right direction by any possible base value (A C G or T/U), and for each base extension a base extension strength is calculated representing an amount of support for extending the reference vertex by each 1-base value, and indicate a left or right direction of the base extension (block 702). In one embodiment, the extension strength may be calculated based at least in part on the number of mapped mated reads that have the same extension and the number of matches and mismatches of those mapped mated reads with the sequence of the vertex being processed,.

The process may partition the reference polynucleotide sequence into reference segments (block 704), to divide the overall computation into shorter segments. In one embodiment, the reference polynucleotide sequence may be partitioned into 50-base reference overlapping or adjacent segments.

Likely variations from the reference are found by finding up to M top base extensions incompatible with the reference vertices in each of the reference segments and whose base extension strength is greater than a first threshold (block 706). In one embodiment, the top two (M=2) base extensions may be used. In one embodiment, each of the base extensions may extend 56 bases past a reference vertex, for example.

The M top base extensions may be used as branch vertices in a partial de Bruijn graph and each branch vertex and direction of extension is input into a recursive procedure that builds the partial de Bruijn graph (block 708).

In a depth-first recursive process, a set of mapped reads is determined that map to the branch vertex and that includes a base extension that extends beyond the branch vertex in the corresponding direction by each possible base value (A C G or T/U), and for each base extension a branch extension strength is calculated representing an amount of support for extending the branch vertex by each base value (block 710).

The process creates new branch vertices from the base extensions having the branch extension strength above a branch extension threshold (block 712).

A new edge is then constructed between the branch vertex and each of the new branch vertices, wherein a chain of the branch vertices connected by one or more edges forms a path through the graph (block 714).

An edge extension strength is calculated for the edges in the path, and the top N edges with the largest edge extension strength above an edge strength threshold are found, and block 722 is performed using each of the top N edges (block 716).

If block 722 fails to find a match between the new branch vertices and the reference vertices, then the top N new edges are input into the recursive procedure (710) as branch vertices (block 718).

If no edges have an extension strength above the strength threshold, the recursive procedure is ended and a failure for the path is returned (block 720).

Each of the new branch vertices is compared to the reference vertices in the partial de Bruijn graph that represent nearby base sequences from the reference, and if the new branch vertex equals any of the base sequences of the reference vertices, the recursive procedure is ended and the path is returned (block 722).

The output of the partial de Bruijn graph procedure may be a file, list, or other data structure containing the local de novo intervals 512. The local de novo intervals 512 may comprise a list of base positions at which the partial de Bruijn graph diverges (branches from the reference.

In one embodiment, the Compute Reference Scores 500 process and the Compute Local De Novo Intervals 501 process may be performed simultaneously and in parallel across the computer cluster 10 so that the process of finding mapped mated reads does not need to be repeated. The results of the reference scores 510 and the local de novo intervals 512 processes computed by individual batches may be consolidated into a single reference scores file and a single local de novo intervals file.

FINDING OPTIMIZATION INTERVALS 502

The processes above find the local areas 300 that are likely to have bases that are changed from the reference. These local areas 300 are further processed as follows. During the Find Optimization Intervals 502 process, individual local areas 300 of likely change represented by the reference scores 510 and the local de novo intervals 512 may be combined to form larger optimization intervals. At a conceptual level, the resulting optimization intervals are identified as areas as likely to contain variations and that require further analysis to determine the likely variations, if any.

The Find Optimization Intervals 502 process considers as candidate optimization intervals the local de novo intervals 512, and the reference scores 510 associated with a high probability ratio, e.g., high $L(G)$. The candidate optimization intervals that overlap or are less than a threshold base distance apart (e.g., less than the length of an arm 210) may be combined to form the optimization intervals. However, some of the optimization intervals may become too long, and optimizing such optimization intervals could become too expensive. In one embodiment, these optimization intervals are therefore no-called *a priori.*

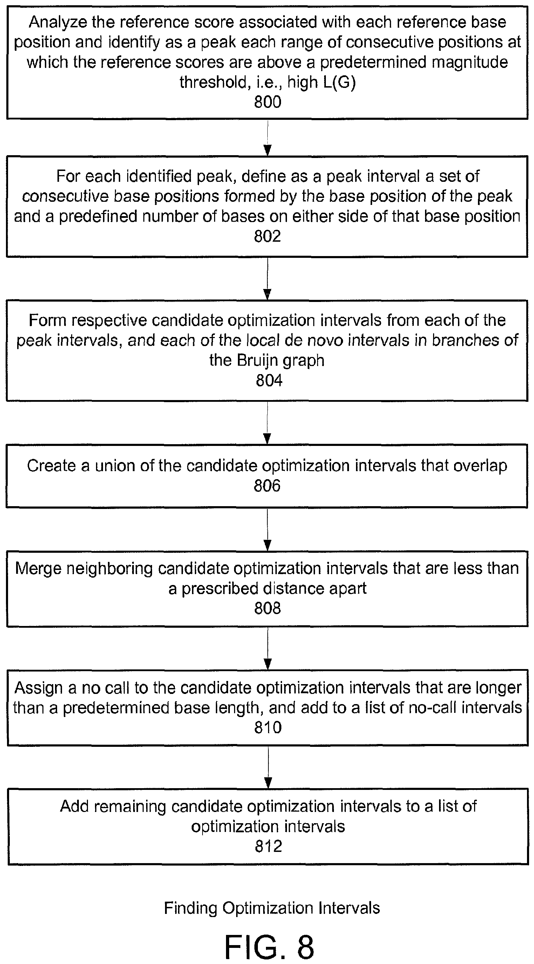

Figure 8 is a flow diagram illustrating a process for finding optimization intervals according to one exemplary embodiment. The Find Optimization Intervals 502 process may include analyzing the reference score associated with each reference base position and identifying as a peak each range of consecutive position at which the reference scores are above a predetermined magnitude threshold, i.e., high $L(G)$ (block 800). In one embodiment, the magnitude threshold for high $L(G)$ may be -10 db, for example.

For each identified peak, a peak interval may be defined as a set of consecutive base positions formed by the base position of the peak and a predefined number of bases on either side of that base position (block 802). In one embodiment, a peak interval may comprise the peak itself plus 3 additional bases on each side.

The Find Optimization Intervals 502 process may form respective candidate optimization intervals from each of the peak intervals and each of the local de novo intervals found in branches of the Bruijn graph (block 804).

The Find Optimization Intervals 502 process may also create respective unions of the candidate optimization intervals (i.e., individual peak intervals and/or local de novo intervals) that overlap (block 806). Neighboring candidate optimization intervals that overlap or are less than a prescribed distance apart may be merged (block 808). In one embodiment, neighboring candidate optimization intervals are merged if they are less than the length of a sequence arm 210 apart. In one specific example, neighboring candidate optimization intervals may be merged if they are within a distance of 20 bases.

A no call is assigned to the candidate optimization intervals that are longer than a predetermined base length, and such candidate optimization intervals are added to a list of no call intervals (block 810). All remaining candidate optimization intervals are added to a list of optimization intervals (block 812). The process of assigning a no call to the candidate optimization intervals is an additional step that reduces computational cost, but is not mathematically required. In an alternative embodiment, the assignment of a no call could be skipped, or equivalently, the predetermined base length might be set to a value so high that no candidate optimization intervals are no-called.

The Find Optimization Intervals 502 process may output a file, list or other data structure containing the optimization intervals 514. Each row of the file may contain positions of the beginning and end of the optimization interval 514. This can include chromosome identification and begin/end positions, or equivalent information. The optimization intervals may also include the initial hypotheses of each 1-base variation generated during the Compute Reference Scores 500 process, or alternative sequences corresponding to de Bruijn graph paths generated during the Compute Local De Novo Intervals 501 process.

The Find Optimization Intervals 502 process may also output a file, list or other data structure containing no-call intervals 516, which are the optimization intervals that are longer than a predetermined base length and that have been identified as too difficult to process (typically because of low read coverage). No optimization is performed on these intervals, and these intervals are eventually no-called in the final variations file 32. Each no-call interval 516 may be identified in a manner similar to what is done for optimization intervals. In one embodiment, the no-call intervals could eventually be analyzed in a separate fashion.

In one embodiment, the Find Optimization Intervals 502 process may be performed in parallel across the computer cluster 10 and the optimization intervals 514 computed by individual batches may be consolidated into a single file of optimization interval and a single file of no-call intervals 516.

OPTIMIZATION

The optimization intervals 514 correspond to portions of the reference where the sample genome of interest may have alternative sequence(s), termed variations. The preceding steps provide not only the intervals but tentative initial hypotheses regarding the specific variations in each interval; these initial hypotheses may be the best combination of bases identified during the Compute Reference Scores 500 process, or they may be the sequence(s) corresponding to the novel path(s) found during extension of the Compute Local De Novo Intervals 501.

The Generate Hypotheses and Optimize using the Bayesian Formulation and the De Bruijn Graph 504 (Hereinafter, Generate Hypotheses and Optimize) process analyzes each optimization interval 514 and attempts to revise these initial hypotheses to improve their fit to the data, i.e., the mapped mated reads 28. The Generate Hypotheses and Optimize 504 process e may output a set of optimized sequence hypotheses of high probability for each of the optimization intervals.

In one embodiment, the optimization 504 stage uses the Bayesian formation 20 in which for each optimization interval, the bases of the initial hypotheses in the optimization interval are iteratively changed one base at a time, including inserted and deleted bases, until the probability increases to a maximum. The Bayesian formation 20 process works for its intended purpose, but in some cases, the probability of these revised sequence hypotheses can't be increased using one base variations. In one embodiment, the probability is increased in these cases by using larger variations, e.g., five bases.

According to one embodiment, the de Bruijn graph 22 may be used to generate additional starting sequence hypotheses as variation directions for the Bayesian formation of the optimization 504 stage. Bayesian scoring involves computing a probability ratio $L(G)$ for each sequence hypothesis.

In one embodiment, the optimization process computes $L(G)$ as:

$$L(G) = log \ (P_H / P_{Ref}),$$

where $P_H$ is a probability of the revised sequence hypothesis in the active interval and $P_{ref}$ is a probability of the reference, and where the set of mapped mated reads in the active interval are used during calculation of the probabilities at each base position. The revised sequence hypothesis is retained that results in a maximum probability ratio. This revised sequence hypothesis is considered the optimum hypothesis for that particular active interval. This process of finding the best one-base substitution from the current hypothesis, and choosing the resulting updated hypothesis as the basis for the next round, is repeated for each or a subset of the initial sequence hypotheses.

In a further embodiment, the logarithmic likelihood ratio $L(G)$ may be expressed, (described more fully below) as:

$$L(G) = log \ \frac{P(G|MtdRds)}{P(G_0|MtdRds)}.$$

Conceptually, the Bayesian formulation outlined above allows polynucleotide sequence variation and reassembly to be represented as an optimization problem of finding the genome G that gives the largest possible $L(G)$. A simple procedure could use greedy optimization that could be described algorithmically as follows:

Start by setting $G = G_0$ and compute $L(G)$.
Apply a small change to $G$ and recompute $L(G)$.

If $L(G)$ increased relative to the previous value, keep the small change applied. Otherwise, undo the change.
Keep iterating until none of the small changes applied cause a further increase in $L(G)$.
The final G obtained in this way is a best guess at the sample genome.
However, without refinements such a procedure would run into a number of computational problems. One problem is that every time $G$ is updated, an efficient way to find mated reads 200 that have good mappings on $G$ is required. This mapping requires some form of mated read indexing, which can be problematic in terms of memory requirement. Another problem is that the computation requires random access to the base reads and the reference scores of the $N_D$ mated reads, which again results in large memory requirements. It is also expensive to recompute all the mappings at each iteration, so data structures that can be incrementally updated as the small changes are applied to $G$ are needed. This again may create a memory problem and may pose a large cost in recomputation.
For these reasons, the exemplary embodiments provide a simpler approach in which the small local areas of the sample 24 in the form of the optimization intervals are analyzed separately from one another during the optimization procedure. When optimizing a current optimization interval, which is considered the active interval, it is assumed that $G$ = $G_0$ everywhere outside the active interval being optimized. With this constraint, the sample polynucleotide sequence $G$ is optimized only in the active interval. In one embodiment, the size of the active interval is kept smaller than the minimum possible mate pair length. With this assumption, for a mated read 200 to have a good mapping to $G$, one arm 210 of the mated read 200 must have a good mapping to $G_0$ approximately one mate pair away from the active interval.
This scheme enables an active interval greedy optimization procedure in which at each iteration, all possible one base changes which affect only the active interval are applied to the current $p$ alleles of $G$. This includes changing an individual base position to a different value, deleting it, or inserting a new base to its right or left. If more than one allele is present, this is done in turn for each of the alleles (leaving the remaining allele(s) unchanged). For each modified $G$ obtained in this way $L(G)$ is computed, as described above. One-base changes that result in the largest $L(G)$ are applied to $G$, and iterated until no further improvement in $L(G)$ can be obtained. The details are stored for all hypotheses for $G$ that have a computed probability within a given factor of the most likely hypothesis found, up to a maximum.
Simulations show that in a significant fraction of the cases, the active interval greedy optimization procedure may converge to the true optimum of $L(G)$ - in other words, the optimization procedure reconstructs correctly the sequence to be assembled, e.g., the sequence of a local interval in a genome. However, in many cases it converges to a local optimum of $L(G)$. For this reason, the exemplary embodiments supplement the active interval greedy optimization with a modified de Bruijn graph process to provide additional starting sequence hypotheses (seeds) for the optimization procedure. This significantly increases the success rate of the optimization procedure. In an alternative embodiment, many other approaches to optimization can be used that reduce the impact of local optima, such as simulated annealing or genetic algorithms.
To set up the optimization procedure, a set of mated reads having at least one arm that maps near (and possibly overlaps) the active interval with a distance, arm and strand, such that the mated arm can contribute to assembly in the active interval is identified. In one embodiment, this set of mated reads is used to perform a de *novo* assembly of the sequence in the active interval.

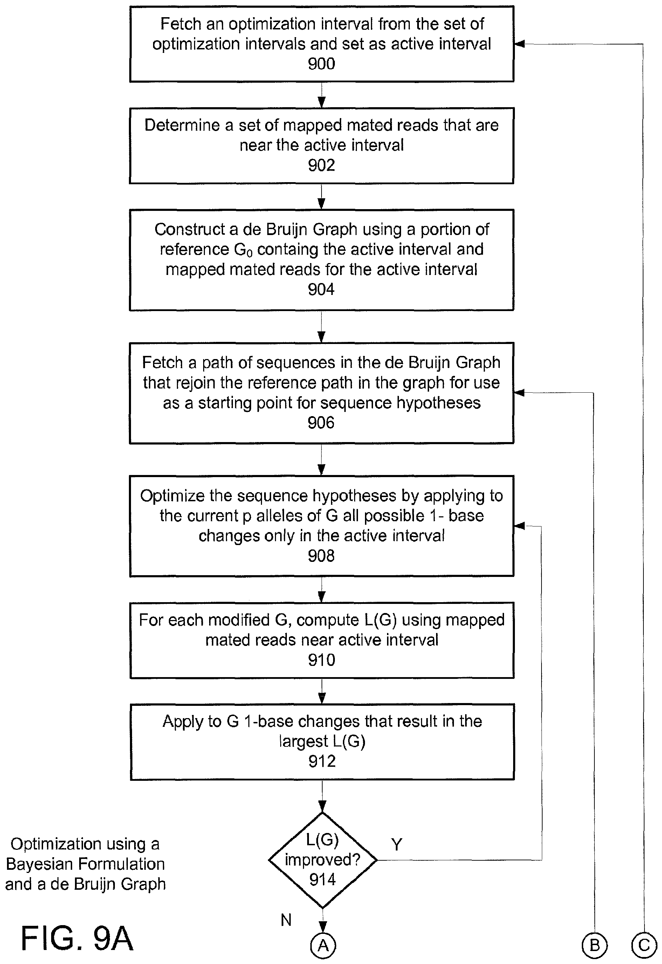

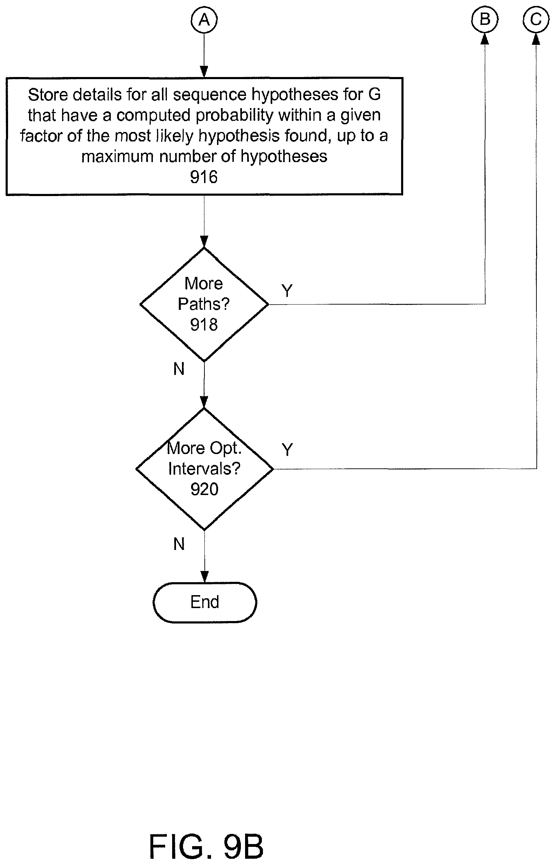

Figures 9A and 9B are flow diagrams illustrating the process for generating hypotheses and optimizing using a Bayesian formulation and a de Bruijn graph. The process may include fetching an optimization interval from the set of optimization intervals and setting the optimization interval as the active interval (block 900).

A set of mapped mated reads that are near the active interval are determined (block 902). According to one embodiment, a mated read is considered to map to the active interval when the reads 204 of one of the mate read arms 210 can be derived from a sequence within the active interval in an orientation consistent with the arm 210. There is no requirement that the second arm be mapped, or if mapped, that it be mapped in or near the active interval.

A de Bruijn graph is constructed using a portion of the reference $G_0$ containing the active interval and the set of mapped mated reads for the active interval (block 904). In an alternative embodiment, the de Bruijn graph can be constructed using a portion of the reference $G_0$ containing the active interval plus a sequence on either side of the active interval of some predetermined length). In one embodiment, the process may include initializing the de Bruijn graph with reference vertices that each represent a base sequence of N contiguous bases from the reference genome, and where each base sequence overlaps an immediately adjacent base sequence, e.g., sharing all but by 1 base. The path of the reference vertices through the graph is referred to as a reference path. The mapped mated reads may be used to create branches from the reference path.

A path of one or more sequences in the de Bruijn graph that rejoins the reference path is fetched for use as a starting point for sequence hypotheses (block 906). The sequence hypotheses are optimized by applying to the current $p$ alleles of $G$ all possible 1-base changes only in the active interval (block 908). For each modified $G$, $L(G)$ is computed using the mapped mated reads near the active interval (block 910). All one-base changes that result in the largest $L(G)$, e.g., greater than a threshold, are applied to $G$ (Block 912). It is then determined whether $L(G)$ is improved (block 914).

If $L(G)$ is improved then the process continues optimizing sequence hypotheses (block 908). If it is determined that $L(G)$ is not improved (block 914), then continuing with Figure 9B, details are stored for all optimized sequence hypotheses for G that have a computed probability within a given factor of the most likely hypothesis found, up to a maximum number of optimized sequence hypotheses (block 916). In one embodiment, at least a given number of optimized sequence hypotheses are saved (or all optimized sequence hypotheses if the number does not reach a predetermined threshold), plus all additional hypotheses within the given factor of probability, and the best hypothesis worse than this factor.

It is then determined whether more paths exist in the de Bruijn graph (block 918). If so, then the process continues by fetching another path (block 906). If no more paths exist in the de Bruijn graph, then it is determined whether there are more optimization intervals (block 920). If so, the process continues with fetching another optimization interval (block 900). Otherwise, the process ends.

Accordingly, the optimization process is supplemented with a procedure in which local de novo assemblies are calculated in the optimization intervals to generate multiple plausible seed sequences, which in turn, are used to drive the optimization process towards a global optima. The local de novo assembly of the present embodiment has been substantially modified from prior de Bruijn graph approaches that are used on contiguous reads in order to accommodate variably gapped reads, as described below. The set of mated read arms used for each local de novo assembly is selected from the mated reads that mapped to the reference one mate pair away from the active interval. This seeding procedure may result in an optimization process that is more resilient to the existence of local optima in the $P(G|MtdRds)/P(G_0|MtdRds)$ landscape. Also, although genomic areas are processed one at a time, joint probabilities of pairs of distant variations are computed, resulting in a substantial reduction a false positive in areas of segmental duplication or other repeated elements in the reference, as described below.

The output of the optimization process 504 is a hypotheses file 518 that contains for each of the selected optimization intervals that were optimized, a list of the most likely optimized hypotheses for the $p$ alleles of $G$ in the active region, together with the corresponding computed $L(G)$, the hypothesis's computed probability ratio relative to the reference polynucleotide sequence.

In one embodiment, the hypotheses file 518 may contain a block for each optimization interval on which an optimization was performed, where the block contains: 1) Positions of the beginning and end of the optimization interval the block refers to, which can include a chromosome identification and begin/end positions, or equivalent information; 2) A list of the most likely hypotheses encountered during the optimization process for this optimization interval. The file may store for each hypothesis, the sequence that replaces the reference sequence for each allele, and $L(G) = log(P_H/P_{Ref})$.

The optimization process may be performed in parallel across the computer cluster 10. The batch results from the computer cluster 10 may be combined into a file, list or other data structure of hypotheses 518. In one embodiment, a mated read support file containing $\Delta(mated\ read)$ information to be used to compute correlations between pairs of distant variations, described further below.

EXTRACTING CALLS

The Extract Calls 506 process infers variations in the bases of the mated reads of the sample polynucleotide sequence in relation to the reference polynucleotide sequence at specific locations by analyzing for each optimization interval the list of optimized hypotheses stored in the hypothesis file 518 and making a decision as to which parts of the top hypothesis are of sufficient confidence to be asserted. The regions in question are those that are consistent with all other top hypotheses for the optimization interval, where the top hypotheses are those with computed probabilities greater than the probability of the most likely hypothesis divided by a threshold, which can be set around a factor of 1000, i.e., 30 dB). More specifically, variations found consistently in the top hypotheses are found and each variation is scored based on the likelihood ratio of the top hypothesis versus the best hypothesis inconsistent with that variation. Areas in which the most likely hypotheses gave contradicting results are no-called

In many cases, the optimization process 504 may result for many of the optimization intervals with a single optimized hypothesis that is computed to be much more likely than all other hypotheses, and which can then confidently be called to describe the correct sequence in the active interval. However, it often happens that an optimization interval may have many similar hypotheses that are all within a small probability factor of the most likely hypothesis. In such cases, making a call on the sequence of $G$ in the optimization interval is more difficult. For example, consider an example where the most likely hypotheses stored are as an example shown in Table I:

| Allele | $L(G)$ (dB) |
| --- | --- |
| ACGATACGAGTAGAAAAAAACTATA | 1000 |
| ACGATACGAGTAGAAAAAACTATA | 995 |
| ACTATACGAGTAGAAAAAACTATA | 0 |

TABLE I

Here, in an example using a haploid genome, the allele corresponding to the reference sequence $G_0$ is provided in bold in the last row of the Table I. Two of the most likely hypotheses generated for the allele are shown in rows 1 and 2 of Table I. Both of the two most likely hypotheses have a SNP at a position highlighted in grey, in which reference base value T is replaced by G in both hypotheses. However, the first hypothesis also has an insertion of an extra A in a homopolymer run. Therefore, the confidence of the existence of the SNP is high; but the existence of the insertion is questionable because even though the insertion is present in the most likely hypothesis, it is not present in the second hypothesis, which is only 5 dB (a factor of 3) less likely than the first hypothesis. Therefore, the desired outcome in this case would be to call the SNP with a score of 1000 dB, but to no-call the region with the homopolymer run.

The Extract Calls 506 process examines for each optimization interval, all hypotheses within a given variation score threshold of the most likely hypothesis listed for the optimization interval, and outputs a set of most likely variations. The Extract Calls 506 process examines the set of most likely hypotheses and finds common features that are present in each of the hypothesis in the set of most likely hypotheses. In one embodiment, inconsistencies between the most likely hypotheses may also be found. The common features that are found can be called confidently within a threshold are stored as respective variation calls 32. On the other hand, inconsistencies between each of the hypotheses in the set of the most likely hypotheses are stored as respective no-call regions. In one embodiment, the variation score threshold can be progressively lowered to provide first, calls for the most confident portion of a region (with highest score), and subsequently calls for less confident portions of the region (with lower scores).

The output of the Extract Calls 506 process is a file, list, or other data structure containing a list of variation calls 32, each describing how base intervals in the resequenced sample polynucleotide sequence are observed to differ from the reference polynucleotide sequence at specific locations. For each variation call, the Extract Calls 506 process may store for the optimization interval where the variation takes place, a chromosome identification and begin/end positions, or equivalent information, and the called sequence for each allele (two alleles in most cases).

In one embodiment, the file of variation calls 32 can also contain a list of the no-called regions, each described in a way similar to the variation calls 32. In another embodiment, the no called intervals may be stored in a separate file. The resequenced polynucleotide sequence is assumed to be equal to the reference polynucleotide sequence outside the base intervals corresponding to the called variations 32 and the no-called regions or alleles.

BAYESIAN FORMULATION

This section describes details of the Bayesian formulation 20. The Bayesian formulation 20 is dependent on the mated read 200 mapping process. Once the position $x$, strand $s$, allele $a$, and gap values $g$ for a mated read are fixed, each base position in at least one arm 210 of the mated read 200 is mapped to a base position in the reference 24 by a mapping $M(x, s, g, a)$, creating a mapped mated read 28. The mapping M is a function that, given a mated read base position $i$, returns the corresponding position in the reference 24 $M(x, s, g, a, i)$ or, with simplified notation, $M(i)$. The events that generate the gap values $g$ are assumed to be uncorrelated from those that generate the position $x$ and strand $s$, and therefore the probability of each mapping is given by:

$$P[M(x, s, g, a)] = P(x, s, a)P(g).$$

In one embodiment, this last assumption may be removed from the formulation. This is useful to allow modeling, for example, in situations where the gap distribution is correlated with a nearby sequence. Such a generalization without the assumption would be straightforward to one skilled in the art.

MATED READ GENERATION PROBABILITY

Once a mapping is fixed, each base position of the mated read corresponds to a base position in the reference 24. In most cases, the base read at that mated read position will equal the corresponding base in the reference. However, due to errors or differences between a reference sequence and the sample under interrogation, this will not always be true.
Based on the error model described above, and under the assumption that the mapping has been fixed, the probability of generating a mated read (*MtdRd*) with base reads $b_i$ is given by:

$$P(MtdRd|G, M) = \prod_i P\{b_i|G[M(i)]\} \prod_i P\{b_i|G[M(i)]\}.$$

Each term in the product can then be computed using the expression for the error matrix $P(b_d|b_g)$ derived above, where $b_d$ is a base as read in the mated read and $b_g$ is a true genomic base being read:

$$P(MtdRd|G, M) = \prod_i \{(1 - \epsilon_i)\delta[b_i, G(M(i))] + [1 - \delta[b_i, G(M(i))]]\epsilon_i/3\}.$$

To summarize in words, each term of the product equals $(1- \epsilon_i)$ it the mated read 200 base is identical to the genome base at that position, and $\epsilon_i/3$ otherwise.
Combining the previous results, and under the further assumption that the base read process is uncorrelated from the mated read 200 generation process, the probability that the sample polynucleotide sequence, e.g., genome $G$, will generate a mated read 200 with base reads $b_i$ is given by:

$$P(MtdRd|G) = \sum_M P(M)P(MtdRd|G, M) = \sum_M P(x, s, a)P(g)P(MtdRd|G, M)$$

Here, the sum is extended over all possible mappings of a mated read 200 to $G$, that is, to all possible values of position $x$, strand $s$, allele $a$, and gap values $g$ for the mated read 200. According to one embodiment, as described below, an approximation is made that allows a significant reduction in the number of terms in the sum.
Once the coverage bias is ignored, the above expression becomes:

$$P(MtdRd|G) = \frac{1}{2N_G} \sum_M P(g)P(MtdRd|G, M).$$

APPLICATION OF BAYES' THEOREM

For purposes of illustrating the exemplary embodiments, and by no means to limit the scope of the embodiments, the input is assumed to be a reference genome $G_0$ and then a sample genome $G$, and an *a priori* estimate of their probability ratio $P_0(G)/P_0(G_0)$ is used. The sample genome G is not an input, but is a desired output of the computation. During optimization, the following equations are applied for many candidate possibilities for $G$ in an effort to find the $G$ with the highest probability.
As a result of Bayes' theorem the observation of a single mated read 200 gives a new estimate for the probability ratio:

$$\frac{P(G|MtdRd)}{P(G_0|MtdRd)} = \frac{P_0(G)}{P_0(G_0)}\frac{P(MtdRd|G)}{P(MtdRd|G_0)}$$

If all mated reads are assumed to be generated independently of each other, the above can be iterated to obtain probability estimates that take into account all of the observed mated reads:

$$\frac{P(G|MtdRds)}{P(G_0|MtdRds)} = \frac{P_0(G)}{P_0(G_0)}\prod_{MtdRds}\frac{P(MtdRd|G)}{P(MtdRd|G_0)}$$

If all *a priori* information is ignored, and thus $P_0(G) = P_0(G_0)$ is assumed for all G's, the previous equation becomes

$$\frac{P(G|MtdRds)}{P(G_0|MtdRds)} = \prod_{MtdRds}\frac{P(MtdRd|G)}{P(MtdRd|G_0)}$$

If the expression for $P(MtdRd|G)$ derived above is plugged in, the result is

$$\frac{P(G|MtdRds)}{P(G_0|MtdRds)} = \left(\frac{N_{G_0}}{N_G}\right)^{N_D}\prod_{MtdRds}\frac{\sum_M P(g)P(MtdRd|G,M)}{\sum_M P(g)P(MtdRd|G_0,M)}$$

The probability ratios may be measured in decibels (dB), each order of magnitude (factor of 10) corresponding to 10 dB. For example, a factor of 1000 corresponds to 30 dB, and a factor of 2 corresponds to approximately 3 dB. This allows expressing large ratios in a convenient way. Decibels are usually used to measure signal to noise ratios, which is appropriate for the present invention because the less likely of the two hypotheses in a ratio can be considered as measurement noise relative to the most likely hypothesis. A ratio r can be expressed in decibels by computing $10log_{10}r$.

INSERTION PENALTY

When $G$ and $G_0$ are substantially similar, the ratio $N_{G_0}/N_G$ (the number of bases in the reference genome divided by the number of bases in the sample genome) is almost exactly equal to 1. So at first sight it would seem that the prefactor in the previous expression can be neglected. However this is not the case because that ratio has an exponent $N_D$ (the number of mated reads from the sample genome), which is a large number. To evaluate the prefactor, it may be set forth as $N_G = N_{G_0} + \Delta N_G$, with $\Delta N_G/N_{G_0} \ll 1$. The prefactor can then be determined as

$$\left(\frac{N_{G_0}}{N_G}\right)^{N_D} = \frac{1}{\left(1 + \Delta N_G/N_{G_0}\right)^{N_D}}$$

The fact that $\Delta N_G/N_{G_0} \ll 1$ allows the writing of an insertion penalty approximation

$$\left(\frac{N_{G_0}}{N_G}\right)^{N_D} = \exp\left(-\frac{N_D}{N_{G_0}}\Delta N_G\right)$$

Therefore it can be rewritten

$$\frac{P(G|MtdRds)}{P(G_0|MtdRds)} = \exp\left(-\frac{N_D}{N_{G_0}}\Delta N_G\right)\prod_{MtdRds}\frac{\sum_M P(g)P(MtdRd|G,M)}{\sum_M P(g)P(MtdRd|G_0,M)}$$

Or, in terms of average coverage per allele c defined above,

$$\frac{P(G|MtdRds)}{P(G_0|MtdRds)} = \exp\left(-\frac{c}{n_D}\Delta N_G\right) \prod_{MtdRds} \frac{\sum_M P(g)P(MtdRd|G,M)}{\sum_M P(g)P(MtdRd|G_0,M)}.$$

This means that each extra base in an allele of $G$ causes a decrease in $P(G|MtdRds)$ by a factor exp $(-c/n_D)$, even if the remaining factor does not change. This "nsertion penalty" expresses the fact that it does not make sense to add extra bases to $G$ unless they have sufficient mated read 200 support. The higher the coverage, the more mated read 200 support is required before a new base can be added to the allele without decreasing $P(G|MtdRds)$. For example, at $c$ = 25, each extra base causes a decrease in by a factor ~1.4 corresponding to 1.5 dB.

If the insertion penalty was not present, $P(G|MtdRds)$ could simply be maximized by adding large amounts of extra sequence to create large numbers of mappings for each mated read 200. The insertion penalty makes this impossible.

In the limit of zero error, the existence of the insertion penalty turns assembly of the polynucleotide sequence into a sort of gapped "shortest common superstring" problem. This is consistent with the fact that the shortest common superstring problem has been used as an abstraction of nucleic acid sequencing.

Handling the insertion penalty in the case where coverage bias is not neglected would require a more complex formulation. This may improve assembly results if coverage bias is high.

For convenience in computation, the previous expression is rewritten in log space:

$$\log\frac{P(G|MtdRds)}{P(G_0|MtdRds)} = -\frac{c}{n_D}\Delta N_G + \sum_{MtdRds} \log\frac{\sum_M P(g)P(MtdRd|G,M)}{\sum_M P(g)P(MtdRd|G_0,M)}$$

For compactness the mated read "weights" are defined as:

$$W(MtdRd,G) = \sum_M P(g)P(MtdRd|G,M)$$

$$W_0(MtdRd) = \sum_M P(g)P(MtdRd|G_0,M).$$

The logarithmic likelihood ratio of relative to $G_0$ is also defined as:

$$L(G) = \log\frac{P(G|MtdRds)}{P(G_0|MtdRds)}.$$

And can be written:

$$L(G) = -\frac{c}{n_D}\Delta N + \sum_{MtdRds}\log\frac{W(MtdRd,G)}{W_0(MtdRd)}.$$

## α APPROXIMATION

The last equation of the previous section allows $L(G)$ to be computed for any hypothetical genome G. The problem, however, is that for each mated read $W(G, MtdRd)$ and $W(G_0)$ need to be computed, which involve sums over all of its possible mappings to $G$ and $G_0$, regardless of the number of mismatches between the mated read and the hypothetical genomic base. Therefore, evaluating $L(G)$ has a prohibitive cost $O(N_D N_G)$ which, for fixed coverage, is also equivalent to $O(N_G^2)$.

On the other hand, for each mated read, only a very small number of mappings are likely to contribute significantly to $W(G, MtdRd)$ and $W(G_0, MtdRd)$. This is due to the multiplying factor $\in/3$ which results from each mismatch between the mated read 200 and the base read 202. As a result, only mappings with small numbers of mismatches give a significant contribution. Mappings with small numbers of mismatches, e.g., 1 to 3, are considered good mappings. If only these good mappings can be identified, the sum can be extended over those mappings only, and the neglected portion of the sum, i.e., the mappings with large numbers of mismatches, approximated with a constant $\alpha$, which is assumed to be the same for all polynucleotide sequence and independent of $G$. With this approximation the following can be written:

$$W(G, MtdRd) = \alpha + \sum_{good\ M} P(g)P(MtdRd|G, M).$$

Although this may be a drastic approximation, its negative effects can be limited with a judicious choice of $\alpha$ and by making a sufficiently inclusive selection of what the "good' mappings are, as discussed in more detail herein. If the number of 'good' mappings per mated read is $0(1)$, the $\alpha$ approximation reduces the computational cost to compute $L(G)$ from quadratic to linear $O(N_G)$. However, this requires ways to neglect mated reads 200 that have too many good mappings, e.g., by using overflow cutoffs during mapping.

The quantity $\alpha$ has the effect of controlling the amount of contribution a single mated read 200 can give to $L(G)$. For a mated read 200 that does not have any good mappings to $G_0$, $W(G_0, MtdRd)=\alpha$. Therefore, if $\alpha$ is chosen to be extremely small, the ratio $W(MtdRd,G)/W_0(MtdRd)$ can become large, and the contribution of that mated read 200 to $L(G)$ can also become large. As $\alpha$ is increased, the maximum contribution a single mated read 200 can give gets smaller.

SNP SCORES (HAPLOID CASE)

Consider a genome $G$ which differs from $G_0$ only by a single nucleotide polymorphism (SNP) at a single haploid base position $i$. To simplify the exposition, assume that $G_0(i) = A$ and $G(i) = C$. In that case, $L(G)$ represents the logarithmic likelihood ratio of the SNP relative to the "null' hypothesis in which the SNP is not present, under the assumption that the two genomes are otherwise identical.

At each haploid base position three values of $L(G)$ can be computed, each corresponding to one of the three possible SNPs at that position. If these "SNP scores" are computed, in most locations values that are large and negative will occur, expressing the fact that the reference is the most likely hypothesis. However, at positions where a SNP exists, the corresponding $L(G)$ will have a positive peak. These peaks already provide a first form of variation calling, because they indicate positions where SNPs are likely to be present. The strength of each peak also offers a measure of the strength of the mated read 200 evidence in favor of the corresponding SNP. This way of detecting SNPs is described in, *e.g.,* the PolyBayes package (Marth GT et al., Nature Genetics 23, 452-456 (1999)), herein incorporated by reference in its entirety.

Unfortunately, however, the presence of other variations in the same region of $G$ can affect the probability of the SNP, if there are mated reads 200 that cover both the SNP and one of the other variations. In other words, the probability of the SNP and the probability of the nearby variations can be correlated. This takes away from the interpretation of $L(G)$ as a direct measure of the likelihood of the existence of the SNP. Correlation issues among nearby variations can easily be taken into account by computing $L(G)$ for hypotheses involving more than just a single SNP.

As an matter of illustration, and not limitation, if it is assumed that $n_A$ concordant mated reads were mapped at base position $i$ with $A$, and $n_C$ discordant mated reads were mapped with $C$, and all of those mated reads have no other good mappings at other locations, and that they all have the same $P(g)$ and the same estimated error rate at that position, then:

$$L(C) = (n_C - n_A) \log \frac{1 - \epsilon}{\frac{\epsilon}{3}} \approx (n_C - n_A) \log \frac{3}{\epsilon},$$

where $C$ is the genome with the SNP and the $\approx$ sign denotes approximate equality to first order in $\in$.

For the sake of illustration, assume $\in = 3\%$. Then $L(C) \approx (n_C - n_A) \times 20$ dB.

In other word each mated read with $A$ contributes 20 dB towards the $A$ hypothesis (reference), and each mated read 200 with $C$ contributes 20 dB towards the $C$ hypothesis (SNP). For example, if 20 $A$'s and 5 $C$'s were obtained, $L(C) \approx -300$ dB. In such a case, the reference hypothesis is overwhelmingly more likely than the SNP hypothesis.

SNP SCORES (DIPLOID CASE)

In the diploid case things are more complex. Consider a diploid base position of $G_0$ and again, for illustration purposes only, assume that $G_0(i) = A$. In a diploid scenario there are two alleles to consider. This means that the number of hypotheses that need to be considered is larger. There are 4 homozygous hypotheses, one of which is the reference while the remaining 3 are the 3 possible homozygous SNPs at that position. There are also 6 heterozygous hypotheses (because hypothesis *AC* and *CA* are equivalent). Of these, 3 are heterozygous SNPs with one base equal to the reference, and 3 are heterozygous SNPs in which both alleles are different from the reference.

The logarithmic likelihood $L(G)$ can be calculated after setting $G$ to each of these 10 hypotheses. In most regions, no SNP is present and therefore all of the 10 values will be large and negative, expressing the fact that the homozygous reference is the most likely hypothesis. The presence of SNPs will be indicated by peaks in one of the 10 scores $L(G)$.

In another specific illustration, $n_A$ concordant mated reads mapped at base position $i$ with $A$, and $n_C$ discordant mated reads mapped with $C$. For the purpose of this illustration, it is assumed that all of those mated reads have no other good mappings at other locations, and that they all have the same $P(g)$ and the same estimated error rate $\epsilon$ at that position.

Now there are three relevant hypotheses: reference ($AA$), heterozygous SNP ($AC$), and homozygous SNP($CC$). For each of the concordant mated reads:

$$\frac{P(MtdRd_A | CC, M)}{P(MtdRd_A | AA, M)} = \frac{\epsilon/3}{1 - \epsilon} \approx \frac{\epsilon}{3}$$

and

$$P(MtdRd_A | AC, M) = \frac{1}{2}[P(MtdRd_A | AA, M) + P(MtdRd_A | CC, M)],$$

from which can be concluded:

$$\frac{P(MtdRd_A | CC, M)}{P(MtdRd_A | AA, M)} = \frac{1}{2}\left[1 + \frac{\epsilon/3}{1 - \epsilon}\right] \approx \frac{1}{2}.$$

Similarly, for each of the $n_C$ discordant mated reads

$$\frac{P(MtdRd_C | CC, M)}{P(MtdRd_C | AA, M)} = \frac{1 - \epsilon}{\epsilon/3} \approx \frac{3}{\epsilon}$$

and

$$P(MtdRd_C | AC, M) = \frac{1}{2}[P(MtdRd_C | AA, M) + P(MtdRd_C | CC, M)],$$

from which can be concluded:

$$\frac{P(MtdRd_C | CC, M)}{P(MtdRd_C | AA, M)} = \frac{1}{2}\left[1 + \frac{1 - \epsilon}{\epsilon/3}\right] \approx \frac{3}{2\epsilon}.$$

Putting this all together, the logarithmic likelihood of the homozygous and heterozygous SNP is:

$$L(CC) = (n_C - n_A)\log\frac{1 - \epsilon}{\epsilon/3} \approx (n_C - n_A)\log\frac{3}{\epsilon}$$

$$L(AC) = n_A \log \left\{ \frac{1}{2} \left[ 1 + \frac{\epsilon/3}{1-\epsilon} \right] \right\} + n_C \log \left\{ \frac{1}{2} \left[ 1 + \frac{1-\epsilon}{\epsilon/3} \right] \right\} \approx n_A \log \frac{1}{2} + n_C \log \frac{3}{2\epsilon}.$$

For $\epsilon$ = 3% this gives approximately:

$$L(CC) = (n_C - n_A) \times 20 \text{ dB}$$

$$L(AC) = -n_A \times 3\text{dB} + n_C \times 17 \text{ dB}.$$

While a single discordant mated read 200 gives a contribution of 17 dB to the heterozygous SNP hypothesis *AC*, a single concordant mated read gives a contribution of only 3 dB to the reference hypothesis AA, relative to *AC*, regardless of error rate. This is a fundamental consequence of the diploid assumption. It expresses the fact that read errors give rise to mated reads similar to the ones that would be generated by a heterozygous hypothesis. This makes diploid calls harder to make than haploid calls, for the same coverage and error rate.

CORRELATION ANALYSIS

The above formulation is used to compute $L(G)$ for a genome $G$ which differs from $G_0$ only in a single small area, called the active interval. In that case, computing $L(G)$ gives information regarding the likelihood of a given variation in the active interval, under the assumption that $G$ and $G_0$ are identical outside the active interval.

However, it is also useful to consider at the same time the possible presence of variations in two separate areas of the genome, *A* and *B*, potentially far away from each other. Specifically, if the two areas are sufficiently separated it is impossible for specific polynucleotide sequence such as those generated with certain empirical operations to have a mapping covering both regions, even partially. The following three hypothetical genomes are then considered:

$G_1$, which is identical to $G_0$ everywhere except in region *A*.
$G_2$, which is identical to $G_0$ everywhere except in region *B*.

$G_{12}$, which is identical to $G_1$ in region *A,* is identical to $G_2$ in region *B*, and identical to $G_0$ everywhere else. In these genomes, $G_1$ contains a hypothetical variation in area *A*, $G_2$ contains a hypothetical variation in region *B*, and $G_{12}$ contains both of those same hypothetical variations.

The computation of $L(G_{12})$ can be performed at little additional cost, if $L(G_1)$ and $L(G_2)$ have already been computed. Defining $\delta_1$ and $\delta_2$ by the equations:

$$W(MtdRd, G_1) = W(MtdRd|G_0) + \delta_1(MtdRd) = W_0(MtdRd) + \delta_1(MtdRd)$$

$$W(MtdRd, G_2) = W(MtdRd|G_0) + \delta_2(MtdRd) = W_0(MtdRd) + \delta_2(MtdRd).$$

It follows that:

$$W(MtdRd, G_{12}) = W_0(MtdRd) + \delta_1(MtdRd) + \delta_2(MtdRd).$$

This is a consequence of the fact that no mappings covering both areas are possible because the two areas are distant from each other. If, during the computation of $L(G_1)$ and $L(G_2)$, the intermediate quantities $\delta_1(MtdRd)$ and $\delta_2(MtdRd)$ are stored for each mated read, $L(G_{12})$ can be computed at little additional computational cost.

The above equations can be further developed to better understand their implications. For this purpose new quantities can be introduced:

$$\Delta_1(MtdRd) = \frac{\delta_1(MtdRd)}{W_0(MtdRd)}$$

$$\Delta_2(MtdRd) = \frac{\delta_2(MtdRd)}{W_0(MtdRd)}$$

which allows

$$W(MtdRd, G_1) = W_0(MtdRd)[1 + \Delta_1(MtdRd)]$$

$$W(MtdRd, G_2) = W_0(MtdRd)[1 + \Delta_2(MtdRd)]$$

$$W(MtdRd, G_{12}) = W_0(MtdRd)[1 + \Delta_1(MtdRd) + \Delta_2(MtdRd)].$$

For the purpose of this computation, the insertion penalty can be neglected:

$$L(G_1) = \sum_{MtdRds} \log \frac{W(MtdRd, G_1)}{W_0(MtdRd)} = \sum_{MtdRds} \log[1 + \Delta_1(MtdRd)]$$

$$L(G_2) = \sum_{MtdRds} \log \frac{W(MtdRd, G_2)}{W_0(MtdRd)} = \sum_{MtdRds} \log[1 + \Delta_2(MtdRd)]$$

$$L(G_{12}) = \sum_{MtdRds} \log \frac{W(MtdRd, G_{12})}{W_0(MtdRd)} = \sum_{MtdRds} \log[1 + \Delta_1(MtdRd) + \Delta_2(MtdRd)].$$

The $L(G_{12})$ is written as a sum

$$L(G_{12}) = L(G_1) + L(G_2) + C_{12},$$

which is a definition of the correlation term $C_{12}$. In the most common situation in which no correlation is present, $L(G_1)$ and $L(G_2)$ are positive while $C_{12}$ is negligibly small, and therefore $L(G_{12})$ is greater than both $L(G_1)$ and $L(G_2)$. This means that the most likely hypothesis is that both variations are present. However, if $C_{12}$ is negative, this is no longer necessarily the case, and it is possible for $L(G_{12})$ to be less than the greater of $L(G_1)$ and $L(G_2)$. In that case, even though each of the two variations is computed to be more likely to exist than not, the most likely possibility is that only one of them exists.

$C_{12}$ can become significantly negative in certain circumstances. From the equations above

$$C_{12} = \sum_{MtdRds} \log \frac{1 + \Delta_1(MtdRd) + \Delta_2(MtdRd)}{[1 + \Delta_1(MtdRd)][1 + \Delta_2(MtdRd)]},$$

or

$$C_{12} = \sum_{MtdRds} \log \left\{ 1 - \frac{\Delta_1(MtdRd)\Delta_2(MtdRd)}{[1 + \Delta_1(MtdRd)][1 + \Delta_2(MtdRd)]} \right\}$$

It is clear that any mated read for which $\Delta_1(MtdRd) = 0$ or $\Delta_2(MtdRd) = 0$ gives no contribution to $C_{12}$. A mated read can significantly contribute to $C_{12}$ only if both $\Delta_1(MtdRd)$ and $\Delta_2(MtdRd)$ are significantly non-zero, that is, if that mated read contributes significantly to both $L(G_1)$ and $L(G_2)$. For example, in the interesting case where a mated read gives a positive contribution to both $L(G_1)$ and $L(G_2)$, $\Delta_1(MtdRd)$ and $\Delta_2(MtdRd)$ are both positive and therefore that mated read gives a negative contribution to $C_{12}$. As only pairs of loci need to be considered such that at least one mated read makes a sufficiently strong contribution to each of the two loci (where the required strength is a configurable parameter), it is straightforward to efficiently determine those pairs of intervals that need to be analyzed for correlations.

In summary, the presence of many mated reads which contribute to both variations makes it less likely that both variations actually exist.

OPTIMIZATION

This section describes details of the optimization procedure 504. In one embodiment, the optimization process 504 is enabled in part by a setup phase that stores and sorts the mated read arm mappings as follows. First, the mated read arm mappings to $G_0$ are sorted during mapping in reference order. Once the arm mappings are sorted in reference order, the mappings may be used to identify mated reads 200 that can possibly contribute to the computation of $L(G)$ in a given active interval. Such arm mappings may be referred to as active mapped mated reads.

The mapping records are stored with mapping information, the base reads 204, and the quality scores 214 of the corresponding mated read 200. This eliminates the requirement of the variation caller 18 performing random access on the base reads 204 of the mated read or on their quality scores 214. The variation caller 18 can therefore operate efficiently on computers 12 with a relatively small amount of memory.

Each mapping record also stores $W_0(MtdRd)$, except for its $\alpha$ contribution. This allows computation of $W(MtdRd, G)$ as follows. 1) Subtract from $W_0(MtdRd)$ the contribution due to all mappings partially or totally overlapping the active interval. This can be done at the beginning of the calculation, and provides a contribution to $W(MtdRd,G)$ due to all mappings that do not overlap the active interval. This is the case because $G$ is assumed to be identical to $G_0$ outside the active interval. 2) Add back the contribution due to all mappings on $G$ partially or totally overlapping the active interval. This must be done by computing these mapping explicitly for each $G$. However this is greatly simplified by the fact that at this stage only mappings of the active mapped mated reads need to be searched.

This requires a means to quickly find active mapped mated reads that have a good mapping on $G$ in the active interval. For this purpose, a local mated read index is used to index all active mapped mated read in a way that makes it easy to locate their good mappings to $G$ in the active interval.

The index may be created by extracting groups of bases at suitable positions from each of the active subsets of the active mapped mated read and using them as keys. For example, in the previously described mated read architecture in which each subset consists of 4 contiguous reads of 5, 10, 10, 10 bases respectively, this is done as follows. There is an index with a 6-base key and an index with a 7-base key. For each 10-base read, two index entries are added in the 7-base key index, using the first and last 7 bases of that read. In addition, one entry in the 6-base key index is added, consisting of the first and last 3 bases of the read. The 5-base read is not indexed.

Once the index is constructed, keys can be constructed using appropriate sets of bases from $G$ in or near the active interval. When a match between the key from the active mated read and a key from a reference index is found, the sequence of the active mated read is checked to count the number of mismatches (or no-calls)).

This method allows scanning the active interval of $G$ with the guarantee of finding all active subset mappings to $G$ with a specific number of mismatches or no-calls. Many more mappings with larger number of mismatches are also found, but those with too many mismatches are filtered out to avoid having to compute their negligible contribution to $L(G)$.

The hits obtained in the search are combined with mappings of the corresponding non-active arms to obtain good mappings of a full mated read 200 to G. This scheme allows the efficient computation of $L(G)$ for any genome G which differs from the reference genome only in the active interval-keeping in mind the assumption mentioned above that the active interval must be sufficiently small.

This scheme enables the active interval greedy optimization procedure described above. For the most likely hypothesis found for each active interval, the quantities $\Delta_1(MtdRd)$ are also stored for each contributing mated read. These may be used for the subsequent computation of correlation terms $C_{12}$ for pairs of variations.

LOCAL DE NOVO

As described above, the exemplary embodiment supplements active interval greedy optimization with a modified de Bruijn graph process to provide additional starting points (seeds) for the optimization procedure to drive the optimization process towards a global optimum.

Previously existing assembly methods based on de Bruijn graphs apply to contiguous reads without gaps, and therefore cannot be used directly to perform de *novo* assembly from mated reads having variable gaps. Briefly, existing assembly methods based on de Bruijn graphs include choosing an assembly length, $n_C < l$, where $l$ is the read length. A graph is constructed, in which each vertex corresponds to a sequence of length $n_C$ present in at least one of the reads. A directed edge between vertex $V_1$ and vertex $V_2$ is then created if both of the following conditions are true: 1) The sequence associated with vertex $V_2$ can be obtained from the sequence associated with vertex $V_1$ by removing its first base and adding a new base at the end. This is the definition of an edge of the de Bruijn graph. Associated with such an edge is the sequence of $n_C + 1$ bases consisting of the first $n_C$ bases of the sequence associated with vertex $V_1$ plus the last base of the sequence associated with vertex $V_2$; and 2) There is at least one read containing the sequence that would be associated with the directed edge.

For example, suppose $n_C = 5$ is chosen and that there are the following reads of length 6:

CTACGA TACGAC ACGACT

The three sample reads may be associated with graph edges. An assembled sequence can be obtained by simply following paths in the graph. Heterozygous events and assembly uncertainties may be represented by branches in the graph. Repeats of length greater than $n_C$ manifest themselves as loops-that is, the directed graph is not longer acyclic.

Such a procedure can only used with sequence arms if it is accepted that there are only individual $l$-base reads (e.g., 10). However, in one embodiment, the left and right arms 210A and 210B may each include 4 contiguous reads 204, which in turn, each comprise three 10-base reads and one 5-base read. Therefore, the above is not acceptable as it would have the effect of neglecting the 5 bases in the 5-base read of each arm 210A and 210B and, more important, would not use the information on the relative position of the 10-base reads implied by the presence of 10-base reads in a single arm.

According to one aspect of the exemplary embodiment, the de Bruijn graph procedure as implemented in the de Bruijn graph 22 and the partial de Bruijn graph 22 components has been modified as follows to process variably gapped reads.

For the de Bruijn graph 22, the process may include selecting an assembly length $n_C$ that is greater than a length $l$ of a read, e.g., approximately 30 bases. A graph is initialized with vertices, but not edges, using the reference sequence $G_0$ in the active interval. The graph is configured to comprise sequences of length $n_C$ bases associated to vertices and sequences of $n_C + 1$ bases associated to edges.

In one embodiment, new vertices are added to a priority queue that is ordered by vertex strength, where the vertex strength is based on a number of mapped mated reads 28 that suggest existence of the vertex as well as a quality of their mapping to the vertex. At each step of the recursive procedure, the highest priority vertex is removed from the priority queue and tested for the ability to construct new edges to or from that vertex. The recursive procedure ends when the queue is empty, such that that no additional edges and vertices can be added to the graph, or alternatively when a certain maximum number of vertices have been created.

When finished, paths in the graph along the edges that begin and end at the first and last location in $G_0$ are enumerated. Each path provides a new seed sequence for the optimization procedure. If a total of $n_P$ paths are found, including the path corresponding to the reference sequence in that active interval, there are a total $\binom{n_P}{P}$ combinations of $p$ of the seed sequences, where $p$ is the ploidy in the active interval.

The probability $L(G)$ is computed for each of the combinations of seed sequences. The paths with starting sequence hypotheses having the largest probabilities $L(G)$ (e.g., the top 3) are then used in turn as starting sequence hypotheses for the optimization procedure. In addition, the allele combination consisting of the reference for all p alleles is always also used as a seed. This limits the number of optimizations that have to be performed, which can be important in cases when the de Bruijn graph is complex and $n_P$ is large.

The local *de novo* procedure performed by the de Bruijn graph 22 increases greatly the success rate of the optimization procedure, particularly if long variations from the reference are present in the active interval.

VARIATION CALLING USING VARIATION CORRELATION INFORMATION

During the optimization phase, the quantities $\Delta_1(MtdRd)$ for each mapped mated read that contributes to the most likely hypothesis in each of the active intervals are stored. Using these quantities, $C_{12}$ can be computed, and therefore $L(G_{12})$ can be computed for each pair of called variations. $L(G_{12})$ can be compared with both $L(G_1)$ and $L(G_2)$. If one of these three quantities exceeds the other two by more than a predetermined threshold (usually set at around 30 dB), then the corresponding hypothesis is called. This could mean that one of the two variations is likely to actually not be in existence, and therefore the corresponding region is called equal to the reference. This can be done as a post-processing step, which can result in eliminating some variations from the variations file created as described in the previous section.

In some cases, two of the three quantities are too close to confidently make a choice. This requires some no-called region to be added to the variations file. For example, if $L(G_{12})$ = 200 dB, $L(G_1)$ = 200 dB, $L(G_2)$ - 100 dB, both of the two most likely hypotheses contain the variation in region A, which is therefore still called. However, the variation in region $B$ needs to be no-called because $G_{12}$ and $G_1$ are equally likely.

RECAP

According to one specific embodiment, the variation calling and/or reassembly may be performed relative to a reference human genome ($G_0$), and a resequenced genome ($G_1$) is obtained by iteratively maximizing the resequenced genome's *a posteriori* probability P($G_1$|MtdRds), which accounts for all mated reads obtained.

From Bayes' theorem, and under the simplifying assumption that each mated read is generated independently of all others, the a posteriori probability P($G_1$|MtdRds) satisfies

$$\frac{P(G_1|MtdRds)}{P(G_0|MtdRds)} = \frac{P_0(G_1)}{P_0(G_0)} \prod_{MtdRds} \frac{P(MtdRd|G_1)}{P(MtdRd|G_0)}$$

Here, P($G_0$) and P($G_1$) are *a priori* probabilities. The assumption that all $G_1$'s have the same a priori probability yields

$$\frac{P(G_1|MtdRds)}{P(G_0|MtdRds)} = \prod_{MtdRds} \frac{P(MtdRd|G_1)}{P(MtdRd|G_0)}$$

The conditional probabilities P(MtdRd|$G_1$) are evaluated under the assumption that all positions in the genome, alleles at each genome location, and either the coding or non-coding DNA strand, are all equally likely to generate a mated read. Each mated read can therefore originate from a large number of mappings M, each comprising a hypothesis for the location of each of the contiguous reads that make up the mated read. This hypothesis must be consistent with the known possible values for read gaps, but all possible mappings should in principle be considered, regardless of the number of mismatches between the genome and the mated read. Since all mappings are exclusive events, this gives

$$P(MtdRd|G) = \sum_M P(M)P(MtdRd|G,M)$$

Here, G can be $G_0$ or $G_1$. The summation is over all possible mappings of a mated read to G, and $P(M)$ is the probability of that mapping being realized, given the known distribution of read gap values. $P(MtdRd|G,M)$ is the probability of reading the observed bases once the mapping is fixed.

$P(MtdRd|G,M)$ is evaluated at each position under the assumptions that all errors are base substitutions, errors are uncorrelated, and errors are equally likely at all positions in the genome. An error rate ($\in$) is estimated for each base call from its quality score, using a calibration curve obtained from mapping results.

Under this model, $P(MtdRd|G,M)$ is a product of a factor 1 - $\in$ for each base position in the mated read which agrees with the genome, and a factor $\in/3$ for each position which does not agree.

The above model can be used to evaluate the probability ratio $P(G_1|MtdRds)/P(G_0|MtdRds)$ for any $G_1$. This requires a product over all mated reads and, for each mated read, a sum over all possible mappings, which is computationally prohibitive. However, the vast majority of mappings ($M$) give a negligibly small contribution to $P(MtdRd|G)$. Only mappings with a small number of mismatches contribute significantly, because the factor $\in/3$ for each mismatch reduces the contribution in an exponential fashion.

Thus, to make computation tractable, the approximation

$$W(G, MtdRd) = \alpha + \sum_{good\ M} P(g)P(MtdRd|G,M)$$

is employed, where the summation is applied only to the subset of mappings which were likely to contribute most, and where the contribution of the neglected mappings was approximated as a constant $\alpha$, assumed to be the same for all mated reads. An additional simplification includes only considering possibilities in which $G_0$ and $G_1$ are identical except for a small localized area. This formulation allows efficient computation of $P(G_1|MtdRds)/P(G_0|MtdRds)$ for any $G_1$, and enables an iterative optimization process in which $G_1$ is initially set equal to $G_0$ and is then locally perturbed. The greedy optimization process considered only single-base changes or single-base insertions or deletions, guaranteeing optimality for all variations consisting of a single base. The optimization process works for longer variations as well, but its effectiveness degrades as the deviations from the reference become longer and denser. Recomputing $P(G_1|MtdRds)/P(G_0|MtdRds)$ at each iteration of the optimization process allows guiding of a simple greedy optimization process towards a (local) maximum.

Because conventional optimization processes may degrade as the deviations from the reference become longer and denser, the optimization process of the exemplary embodiment is supplemented with a procedure in which local *de novo* assemblies are performed at selected locations to generate multiple plausible seed sequences, which were used in turn to drive the optimization process towards more global optima. Local *de novo* assembly uses a method similar to the De Bruijn graph approaches to contiguous reads, but with substantial modifications to accommodate variably gapped reads. The pool of mated read arms used for each local *de novo* assembly is selected from mated reads that mapped to the reference one mate pair away from the region of interest. This seeding procedure makes the optimization process much more resilient to the existence of local optima in the $P(G_1|MtdRds)/P(G_0|MtdRds)$ landscape. Also, although genomic areas are processed one at a time, joint probabilities of pairs of distant variations are computed, resulting in a substantial reduction of false positives in regions of segmental duplication.

The above-described reassembly procedure generates a hypothesis file that includes for each localized area of the genome, a set of sequence hypotheses for the $G_1$ alleles in the area, as well as their respective probability ratios relative to the null hypothesis $G_0$. In one embodiment, the process generates a variations file containing, for each reference base position, a single most likely reconstruction of the target genome, together with a quality score. This is accomplished by identifying variations found consistently in the top hypotheses (hypotheses with computed probabilities greater than the probability of the most likely hypothesis divided by a threshold) and scoring each variation based on the likelihood ratio of the top hypothesis versus the best hypothesis inconsistent with that variation. Areas in which the most likely hypotheses gave contradicting results are no-called.

A method and system has been disclosed for calling variations in a sample polynucleotide sequence with respect to a reference polynucleotide sequence. While this invention is satisfied by embodiments in many different forms, as described in detail in connection with preferred embodiments of the invention, it is understood that the present disclosure is to be considered as exemplary of the principles of the invention and is not intended to limit the invention to the specific embodiments illustrated and described herein. For example, the exemplary embodiment can be implemented using hardware, software, a computer readable medium containing executable program instructions, or a combination thereof. Software written according to the present invention is to be either stored in some form of computer-readable medium such as a memory, a hard disk, or a CD/DVD-ROM and is to be executed by a processor.

The application relates to a computer-implemented method for calling variations in a sample polynucleotide sequence with respect to a reference polynucleotide sequence, the method comprising:

executing an application on at least one computer that locates local areas in the reference polynucleotide sequence where a likelihood exists that one or more bases of the sample polynucleotide sequence are changed from corresponding bases in the reference polynucleotide sequence, where the likelihood is determined at least in part based on mapped mated reads of the sample polynucleotide sequence;
generating at least one sequence hypothesis for each of the local areas, and optimizing the at least one sequence hypothesis for at least a portion of the local areas to find one or more optimized sequence hypotheses of high probability for the local areas; and
analyzing the optimized sequence hypotheses to identify a series of variation calls in the sample polynucleotide sequence.

According to a preferred example each of the mated reads comprise variably gapped reads.

According to a preferred example each of the mated reads comprise non-gapped reads.

According to a preferred example the method is further comprising storing in a data repository the reference polynucleotide sequence and mapped mated reads obtained from the sample polynucleotide sequence that are mapped to locations in the reference polynuc-leotide sequence, and performing the method by a variation caller executing in parallel on the plurality of computers in a computer cluster, each of the plurality of computers coupled to the data repository via a network.

According to a preferred example locating the local areas likely to have changed from the reference further comprises computing reference scores using a Bayesian formulation process.

According to a preferred example computing reference scores using the Bayesian formulation process further comprises:

generating a set of initial hypotheses for each base position in the reference polynucleotide sequence by modifying a base value at that position in *p* alleles by all possible 1-base variations;
determining a set of mapped mated reads that are near the current base position of the reference polynucleotide sequence; and
computing reference scores for each base position by computing for each of the initial hypotheses in the corresponding set, a probability ratio $P_v/P_{ref}$, where $P_v$ is a probability of a 1-base variation hypothesis, and $P_{ref}$ is a probability of the base value in the reference polynucleotide sequence, and where the set of mapped mated reads near each base position are used during calculation of the probability ratio at each base position.

According to a preferred example the sample polynucleotide sequence comprises a genome G, and wherein each of the reference scores comprises as a logarithmic likelihood ratio L(G) for each of the hypothesis, where L(G) = log ($P_v/P_{Ref}$).

According to a preferred example the mapped mated reads are generated independently of each other, and probability estimates that take into account all of the mapped mated reads are calculated by

$$\frac{P(G|MtdRds)}{P(G_0|MtdRds)} = \left(\frac{N_{G_0}}{N_G}\right)^{N_D} \prod_{MtdRds} \frac{\sum_M P(g)P(MtdRd|G,M)}{\sum_M P(g)P(MtdRd|G_0,M)}$$

where $N_{G_0}$ represent a number of bases in the reference genome, $N_G$ represents a number of bases in the sample genome, and $N_D$ represents a number of mated reads.

According to a preferred example the method is further comprising representing $\left(\frac{N_{G_0}}{N_G}\right)^{N_D}$ with an insertion penalty approximation, such that each extra base in an allele of *G* causes a decrease in $P(G|MtdRds)$ by a factor exp ($-c/n_D$), where $n_D$ represents a number of bases in each of the mapped mated reads, so that extra bases are not added to *G* unless the extra bases have sufficient mapped mated read support.

According to a preferred example the method is further comprising controlling an amount of contribution a single mated read can give to L(G) by considering only the mappings of the mated read that have a small number of mismatches in a summation of the mated reads during calculation of L(G), and representing the mappings with large numbers of mismatches with a constant that is assumed to be the same for all polynucleotide sequence and independent of *G*.

According to a preferred example locating the local areas likely to have changed from the reference further comprises computing local de novo intervals using the partial de Bruijn graph to find variations beyond single base changes.

According to a preferred example the method is further comprising:

initializing a partial de Bruijn graph with reference vertices created from base sequences from the reference polynucleotide sequence;
for each of the reference vertices, determining a set of mapped mated reads that map to the reference vertex and that include a base extension extending beyond either end of the reference vertex by any possible 1-base value;
calculating for each of the base extensions an extension strength representing an amount of support for extending the reference vertex by each 1-base value based at least in part on a number of mapped mated reads that have the same extension and the number of matches and mismatches of those mapped mated reads with the sequence of the vertex being processed; using the base extensions having a highest the extension strength that are incompatible with the reference vertices as a branch vertices in the partial de Bruijn graph;
computing the extension strength in the direction of the extension for each branch vertex in a depth-first manner in one direction, and creating a new edge and a branch new vertex after each computation from the base extensions having extension strength above a threshold;
if there are no base extensions having the extension strength above the threshold in a path, returning a failure for the path; and
if a new branch vertex is created that is equal to the base sequence of one of the reference vertices and that is consistent with a SNP or short indel, ending the computation and returning the path.

According to a preferred example locating the local areas likely to have changed from the reference further comprises finding optimization intervals by combining individual local areas of likely change represented by the reference scores and the local de novo intervals.

According to a preferred example the method is further comprising:

considering as candidate optimization intervals the local de novo intervals and the reference scores associated with a high probability ratio $P_v/P_{ref}$, where $P_v$ is a probability of a 1-base variation hypothesis, and $P_{ref}$ is a probability of the base value in the reference polynucleotide sequence; and

combining the candidate optimization intervals that overlap or are less than a threshold base distance apart into the optimization intervals.

According to a preferred example the sample polynucleotide sequence comprises a genome $G$, and wherein each of the reference scores comprises as a logarithmic likelihood ratio $L(G)$ for each of the hypothesis, where $L(G) = \log(P_v/P_{Ref})$.

According to a preferred example the optimizing a sequence hypothesis for a local area further comprises:

traversing each base position in an initial hypotheses in the local area and iteratively changing the base with every possible alternative base values including inserted and deleted bases, computing a second probability ratio for each change; and

applying changes to the local area that maximize the second probability ratio..

According to a preferred example the method is further comprising performing the optimization on the local areas separately from one another with an assumption that the sample polynucleotide sequence equals the reference polynucleotide sequence outside of the current local area being optimized, such that the sample polynucleotide sequence is optimized only in the current local area.

According to a preferred example the method is further comprising calculating the second probability ratio as $P_H / P_{ref}$, where $P_H$ is a probability of the sequence hypothesis in a current local area and $P_{ref}$ is a probability of the reference.

According to a preferred example the sample polynucleotide sequence comprises a genome $G$, and wherein each of the reference scores comprises as a logarithmic likelihood ratio $L(G)$ for each of the hypothesis, where $L(G) = \log(P_H/P_{Ref})$.

According to a preferred example the method is further comprising performing the optimization using a combination of a Bayesian formulation and a de Bruijn graph, wherein the de Bruijn graph is used to generate additional starting sequence hypotheses for the Bayesian formation to drive the optimization process towards a global optimum, and wherein the de Bruijn graph that is modified to process variably gapped reads.

According to a preferred example the method is further comprising using a de Bruijn graph that is modified to process variably gapped reads.

According to a preferred example the method is further comprising requiring that a size of the current local area is kept smaller than a minimum possible mate pair length.

According to a preferred example the analyzing each of the sets of sequence hypotheses further comprises inferring variations in the bases of the mapped mated reads of the sample polynucleotide sequence in relation to the reference polynucleotide sequence at a specific

According to a preferred example the method is further comprising: examining for each of the local areas, all hypotheses within a variation score threshold of a most likely hypothesis listed for the local area, producing a set of most likely hypotheses; finding common features that are present in each of the hypotheses in the set of most likely hypotheses; and storing the common features as respective variation calls.

According to a referred example the method is further comprising:

finding inconsistencies between each of the hypotheses in the set of most likely hypotheses; and storing the inconsistencies as respective no-call regions.

According to a referred example the sample polynucleotide sequence comprises a genome (G), the method further comprising reassembling the sample polynucleotide sequence by iteratively maximizing the genome's a posteriori probability P(G/MatedReads), which accounts for all mapped mated reads.

According to a referred example analyzing each of the sets of sequence hypotheses further comprises generating a no call for any part of the hypotheses in the set of sequence hypotheses in which an inconsistency is found.

A further aspect of the application is a system, comprising:

a data repository that stores a reference polynucleotide sequence and mapped mated reads obtained from a sample polynucleotide sequence that are mapped to locations in the reference polynucleotide sequence;

a computer cluster comprising a plurality of computers coupled to the data repository via a network; and
a variation caller executing in parallel on the plurality of computers, the variation caller configured to:

locate local areas in the sample polynucleotide sequence based on the mapped mated reads where one or more bases are likely to have changed from corresponding bases in the reference polynucleotide sequence; optimize a sequence hypothesis for each of the local areas to find a set of sequence hypotheses of high probability for each of the local areas; and
analyze each of the sets of sequence hypotheses to identify a series of variation calls in the sample polynucleotide sequence.

A further aspect of the application is a system, comprising:

a data repository that stores a reference polynucleotide sequence and mapped mated reads obtained from a sample polynucleotide sequence that are mapped to locations in the reference polynucleotide sequence;
a computer cluster comprising a plurality of computers coupled to the data repository via a network; and
a variation caller executing in parallel on the plurality of computers, the variation caller configured to:

perform statistical probability analysis on the reference polynucleotide sequence and on the mapped mated reads based in part on a combination of evidential reasoning performed by a Bayesian formulation and de Bruijn graph based algorithms;
use the statistical probability analysis to identify and call variations detected in the mapped mated reads in relation to the reference polynucleotide sequence; and
output a list of the variations, each describing a manner in which the mapped mated reads are observed to differ from the reference polynucleotide sequence at or near a specific location.

According to a referred example of the system the variations file further includes a list of no-called regions for which variations cannot be called due to computational uncertainties.
According to a preferred example of the system the variations include sequences of deletions, insertions, mutations, polymorphisms, and duplications or rearrangements of one or more bases.
According to a preferred example of the system the variation caller is further configured to use the probability analysis to assemble the sample polynucleotide sequence from the mapped mated reads, where an assembled polynucleotide sequence is based substantially on the reference polynucleotide sequence, but includes the identified variations.
According to a preferred example of the system the computer cluster is configured such that instances of the variation caller executing on different ones of the plurality of computers operate on different portions of the reference polynucleotide sequence and the mapped mated reads in parallel.
A further aspect of the application is a computer-implemented method for calling variations in mapped mated reads obtained from a sample polynucleotide sequence compared to a reference polynucleotide sequence, the method comprising:

performing statistical probability analysis on the reference polynucleotide sequence and on the mapped mated reads based in part on a combination of evidential reasoning performed by a Bayesian formulation and de Bruijn graph based algorithms;
using the statistical probability analysis to identify and call variations detected in the mapped mated reads in relation to the reference polynucleotide sequence; and
outputting a list of the variations, each describing a manner in which the mapped mated reads are observed to differ from the reference polynucleotide sequence at or near a specific location and storing the variation calls in a memory.

According to a preferred example he computer-implemented method is improved by further comprising storing in $\alpha$ data repository the reference polynucleotide sequence and mapped mated reads obtained from the sample polynucleotide sequence that are mapped to locations in the reference polynucleotide sequence, and performing the method by a variation caller executing in parallel on the plurality of computers in a computer cluster, each of the plurality of computers coupled to the data repository via a network.

**Claims**

1. A computer-implemented method for calling variations in mapped mated reads obtained from a sample polynucleotide sequence compared to a reference polynucleotide sequence, the method comprising:

    receiving the reference polynucleotide sequence and mapped mated reads (200), wherein the mapped mated reads are obtained from the sample polynucleotide sequence and are mapped to locations in the reference polynucleotide sequence;
    for each of a plurality of positions on the reference polynucleotide sequence:

        calculating one or more reference scores (510), each reference score for a hypothesis different from the reference polynucleotide sequence at the position and calculated based on the mapped mated reads using a Bayesian formulation (500) for the hypothesis;

    identifying local areas (300) corresponding to positions having a reference score above a threshold;
    using de Bruijn graph-based algorithms for determining graphs to identify local de novo intervals (512), each local de novo interval comprising one or more positions at which the graph diverges from the reference polynucleotide sequence;
    combining the local areas and the local de novo intervals to form optimization intervals (514);
    for each optimization interval:

        using the reference scores or one or more graphs of the optimization interval to generate an initial sequence hypothesis (412); and
        modifying the initial sequence hypothesis to obtain an optimized sequence hypothesis (414) having an increased probability of being correct based on the mapped mated reads that map to the optimization interval;

    using the optimized sequence hypotheses to identify and call variations (32) detected in the mapped mated reads in relation to the reference polynucleotide sequence; and
    outputting a list of the variations, each describing a manner in which the mapped mated reads are observed to differ from the reference polynucleotide sequence at or near a specific location.

2. The method of claim 1, wherein outputting the list of the variations further includes outputting a list of no-called regions for which variations cannot be called due to computational uncertainties.

3. The method of claim 1, wherein the variations include identified sequences of deletions, insertions, mutations, polymorphisms, and duplications or rearrangements of one or more bases.

4. The method of claim 3, further comprising using the optimized sequence hypotheses to assemble the sample polynucleotide sequence from the mapped mated reads, where an assembled polynucleotide sequence is based substantially on the reference polynucleotide sequence, but includes the identified sequences.

5. The method of claim 1, wherein each of the mapped mated reads comprises variably gapped reads.

6. The method of claim 1, wherein each of the mapped mated reads comprises non-gapped reads.

7. The method of claim 1, wherein calculating reference scores using the Bayesian formulation comprises:

> for each base position in the reference polynucleotide sequence:

>> generating a set of initial hypotheses for that base position in the reference polynucleotide sequence by modifying a base value at that base position in $p$ alleles by all possible 1-base variations;
>> determining a set of mapped mated reads that are near that base position of the reference polynucleotide sequence; and
>> computing reference scores for that base position by computing for each of the initial hypotheses in the set of initial hypotheses, a probability ratio $P_V/P_{Ref}$, where $P_V$ is a probability of a 1-base variation hypothesis, and $P_{Ref}$ is a probability of the base value in the reference polynucleotide sequence, and where the set of mapped mated reads near that base position are used during calculation of the probability ratio at that base position;

> wherein the sample polynucleotide sequence comprises a genome $G$, and wherein each of the reference scores comprises as a logarithmic likelihood ratio $L(G)$ for each of the hypothesis, where $L(G) = \log (P_V/P_{Ref})$.

8. The method of claim 6, wherein the mapped mated reads are generated independently of each other, and probability estimates that take into account all of the mapped mated reads are calculated by

$$\frac{P(G|MtdRds)}{P(G_0|MtdRds)} = \left(\frac{N_{G_0}}{N_G}\right)^{N_D} \prod_{MtdRds} \frac{\sum_M P(g)P(MtdRd|G,M)}{\sum_M P(g)P(MtdRd|G_0,M)}$$

> where $N_{G_0}$ represent a number of bases in the reference genome, $N_G$ represents a number of bases in the sample genome, and $N_D$ represents a number of mated reads.

9. The method of claim 7, further comprising representing $\left(\frac{N_{G_0}}{N_G}\right)^{N_D}$ with an insertion penalty approximation, such that each extra base in an allele of G causes a decrease in $P(G|MtdRds)$ by a factor exp ($-c/n_D$), where $n_D$ represents a number of bases in each of the mapped mated reads, so that extra bases are not added to G unless the extra bases have sufficient mapped mated read support, where c is the average coverage per allele.

10. The method of claim 1, wherein computing local de novo intervals uses a partial de Bruijn graph to find variations beyond single base changes, the method further comprising:

> initializing the partial de Bruijn graph with reference vertices created from base sequences from the reference polynucleotide sequence;
> for each of the reference vertices, determining a set of mapped mated reads that map to the reference vertex and that include a base extension extending beyond either end of the reference vertex by any possible 1-base value;
> calculating for each of the base extensions an extension strength representing an amount of support for extending the reference vertex by each 1-base value based at least in part on a number of mapped mated reads that have the same extension and the number of matches and mismatches of those mapped mated reads with the sequence of the vertex being processed; using the base extensions having a highest the extension strength that are incompatible with the reference vertices as a branch vertices in the partial de Bruijn graph;
> computing the extension strength in the direction of the extension for each branch vertex in a depth-first manner in one direction, and creating a new edge and a branch new vertex after each computation from the base extensions having extension strength above a threshold;

> if there are no base extensions having the extension strength above the threshold in a path, returning a failure for the path; and
> if a new branch vertex is created that is equal to the base sequence of one of the reference vertices and that is consistent with a SNP or short indel, ending the computation and returning the path.

11. The method of claim 1, wherein combining the local areas and the local de novo intervals to form optimization intervals includes:

considering as candidate optimization intervals the local de novo intervals and the reference scores associated with a high probability ratio $P_v/P_{Ref}$ that exceeds the threshold, where $P_v$ is a probability of a 1-base variation hypothesis, and $P_{Ref}$ is a probability of the base value in the reference polynucleotide sequence; and combining the candidate optimization intervals that overlap or are less than a threshold base distance apart into the optimization intervals;

wherein the sample polynucleotide sequence comprises a genome $G$, and wherein each of the reference scores comprises as a logarithmic likelihood ratio $L(G)$ for each of the hypothesis, where $L(G) = \log (P_v/P_{Ref})$.

12. The method of claim 1 wherein modifying the initial sequence hypothesis to obtain an optimized sequence hypothesis comprises:

traversing each base position in an initial hypotheses in the optimization interval and iteratively changing the base with every possible alternative base values including inserted and deleted bases, computing a probability ratio for each change; and
applying changes to the initial hypotheses that maximize the probability ratio.

13. A system, comprising:

a data repository (14) that stores a reference polynucleotide sequence and mapped mated reads obtained from a sample polynucleotide sequence that are mapped to locations in the reference polynucleotide sequence;
a computer cluster (10) comprising a plurality of computers (12) coupled to the data repository via a network; and
a variation caller (18) executing in parallel on the plurality of computers, the variation caller configured to perform the method in any one of claims 1-12.

14. The system of claim 13 wherein the computer cluster is configured such that instances of the variation caller executing on different ones of the plurality of computers operate on different portions of the reference polynucleotide sequence and the mapped mated reads in parallel.

15. An executable software product stored on a computer-readable medium containing program instructions for performing the method in any one of claims 1-12.


**Patentansprüche**

1. Computer-implementiertes Verfahren zum Abrufen von Variationen in gemappten gepaarten Ablesungen, die von einer Probenpolynucleotidsequenz durch Vergleich mit einer Referenzpolynucleotidsequenz erhalten wurden, wobei das Verfahren umfasst:

Empfangen der Referenzpolynucleotidsequenz und der gemappten gepaarten Ablesungen (200), wobei die gemappten gepaarten Ablesungen von der Probenpolynucleotidsequenz erhalten sind und auf Orte in der Referenzpolynucleotidsequenz gemappt sind;
für jede einer Vielzahl von Positionen auf der Referenzpolynucleotidsequenz:

Berechnen eines oder mehrerer Referenzwerte (510), wobei jeder Referenzwert zu einer Hypothese gehört, die von der Referenzpolynucleotidsequenz an der Position verschieden ist und die berechnet ist basierend auf den gemappten gepaarten Ablesungen unter Verwendung einer Bayes' sehen Formulierung (500) für die Hypothese;

Identifizieren lokaler Bereiche (300), die zu Positionen mit einem Referenzwert oberhalb eines Schwellwertes korrespondieren;
Verwenden von auf de-Bruijn-Graphen basierenden Algorithmen zum Bestimmen von Graphen zum Identifizieren von lokalen de-novo-Intervallen (512), wobei jedes lokale de-novo-Intervall eine oder mehrere Positionen umfasst, an denen der Graph von der Referenzpolynucleotidsequenz abweicht;
Kombinieren der lokalen Bereiche und der lokalen de-novo-Intervalle, um Optimierungsintervalle (514) zu bilden;
für jedes Optimierungsintervall:

Verwendung der Referenzwerte oder eines oder mehrerer Graphen des Optimierungsintervalls, um eine initiale Sequenzhypothese (412) zu erzeugen; und
Modifizieren der initialen Sequenzhypothese, um eine optimierte Sequenzhypothese (414) zu erhalten, die eine erhöhte Wahrscheinlichkeit aufweist, richtig zu sein, basierend auf den gemappten gepaarten Ablesungen, die auf das Optimierungsintervall mappen;

Verwenden der optimierten Sequenzhypothesen, um Variationen (32) zu identifizieren und abzurufen, die in den gemappten gepaarten Ablesungen in Bezug auf die Referenzpolynucleotidsequenz detektiert werden; und
Ausgeben einer Liste der Variationen, wobei jede eine Art und Weise beschreibt, in der die gemappten gepaarten Ablesungen als von der Referenzpolynucleotidsequenz an oder in der Nähe eines bestimmten Ortes abweichend beobachtet werden.

2. Verfahren nach Anspruch 1, wobei das Ausgeben der Liste der Variationen des Weiteren ein Ausgeben einer Liste von nicht-abgerufenen Regionen beinhaltet, für die Variationen aufgrund von rechnerischen Unsicherheiten nicht abgerufen werden können.

3. Verfahren nach Anspruch 1, wobei die Variationen identifizierte Sequenzen von Deletionen, Einfügungen, Mutationen, Polymorphismen, und Duplizierungen oder Umorientierungen von einer oder mehreren Basen umfassen.

4. Verfahren nach Anspruch 3, des Weiteren umfassend ein Verwenden der optimierten Sequenzhypothesen, um die Probenpolynucleotidsequenz aus den gemappten gepaarten Ablesungen zusammenzufügen, wobei eine zusammengefügte Polynucleotidsequenz im Wesentlichen auf der Referenzpolynucleotidsequenz basiert, aber die identifizierten Sequenzen umfasst.

5. Verfahren nach Anspruch 1, wobei jede der gemappten gepaarten Ablesungen variabel beabstandete Ablesungen umfasst.

6. Verfahren nach Anspruch 1, wobei jede der gemappten gepaarten Ablesungen nicht-beabstandete Ablesungen umfasst.

7. Verfahren nach Anspruch 1, wobei das Berechnen von Referenzwerten unter Verwendung der Bayes'schen Formulierung umfasst:

für jede Basenposition in der Referenzpolynucleotidsequenz:

Erzeugen eines Satzes von initialen Hypothesen für diese Basenposition in der Referenzpolynucleotidsequenz durch Modifizieren eines Basenwertes an dieser Basenposition in $p$ Allelen durch alle möglichen 1-Basenvariationen;
Bestimmen eines Satzes von gemappten gepaarten Ablesungen, die nahe dieser Basenposition der Referenzpolynucleotidsequenz sind; und
Berechnen von Referenzwerten für diese Basenposition durch Berechnen eines Wahrscheinlichkeitsverhältnisses $P_V/P_{Ref}$ für jede der initialen Hypothesen in dem Satz von initialen Hypothesen, wobei $P_V$ eine Wahrscheinlichkeit einer 1-Basenvariationshypothese ist, und $P_{Ref}$ eine Wahrscheinlichkeit des Basenwertes in der Referenzpolynucleotidsequenz ist, und wobei der Satz von gemappten gepaarten Ablesungen nahe dieser Basenposition während der Berechnung des Wahrscheinlichkeitsverhältnisses an dieser Basenposition verwendet wird;

wobei die Probenpolynucleotidsequenz ein Genom $G$ umfasst, und wobei jeder der Referenzwerte ein logarithmisches Wahrscheinlichkeitsverhältnis $L(G)$ für jede der Hypothesen umfasst, wobei $L(G) = \log(P_V/P_{Ref})$.

8. Verfahren nach Anspruch 6, wobei die gemappten gepaarten Ablesungen unabhängig voneinander erzeugt werden, und wobei Wahrscheinlichkeitsschätzungen, die alle der gemappten gepaarten Ablesungen berücksichtigen, berechnet werden durch

$$\frac{P(G|MtdRds)}{P(G_0|MtdRds)} = \left(\frac{N_G}{N_C}\right)^{N_D} \prod_{MtdRds} \frac{\sum_M P(g)P(MtdRd|G,M)}{\sum_M P(g)P(MtdRd|G_0,M)}$$

wobei $N_{G_0}$ eine Anzahl von Basen in dem Referenzgenom repräsentiert, $N_G$ eine Anzahl von Basen in dem Probengenom repräsentiert, und $N_D$ eine Anzahl von gepaarten Ablesungen repräsentiert.

9. Verfahren nach Anspruch 7, des Weiteren umfassend ein Repräsentieren von $\left(\frac{N_{G_0}}{N_G}\right)^{N_D}$ mit einer Einfügungsstrafenapproximation, so dass jede zusätzliche Base in einem Allel von $G$ eine Abnahme in $P(G|MdRds)$ mit einen Faktor $\exp(-c/n_D)$ bewirkt, wobei $n_D$ eine Anzahl von Basen in jeder der gemappten gepaarten Ablesungen repräsentiert, so dass zusätzliche Basen nicht zu $G$ addiert werden, außer wenn die zusätzlichen Basen eine ausreichende Unterstützung durch gemappte gepaarte Ablesungen haben, wobei $c$ die durchschnittliche Abdeckung pro Allel ist.

10. Verfahren nach Anspruch 1, wobei das Berechnen lokaler de-novo-Intervalle einen partiellen de-Bruijn-Graphen verwendet, um Variationen über einzelne Basenänderungen hinaus zu finden, wobei das Verfahren des Weiteren umfasst:

Initialisieren des partiellen de-Bruijn-Graphen mit Referenzvertexen, die aus Basensequenzen aus der Referenzpolynucleotidsequenz erzeugt wurden;
für jeden der Referenzvertexe, Bestimmen eines Satzes von gemappten gepaarten Ablesungen, die auf den Referenzvertex mappen und die eine Basenerstreckung umfassen, die sich über eines von beiden Enden des Referenzvertex hinaus durch jeden möglichen 1-Basenwert erstreckt;
Berechnen, für jede der Basenerstreckungen, einer Erstreckungsstärke, die einen Betrag an Unterstützung zum Erstrecken des Referenzvertexes durch jeden 1-Basenwert repräsentiert, basierend zumindest teilweise auf einer Anzahl von gemappten gepaarten Ablesungen, die die gleiche Erstreckung haben, und der Anzahl an Übereinstimmungen und Nichtübereinstimmungen dieser gemappten gepaarten Ablesungen mit der Sequenz des Vertexes, der verarbeitet wird;
Verwenden der Basenerstreckungen, die die höchste mit den Referenzvertexen inkompatible Erweiterungsstärke aufweisen, als Verzweigungsvertexe in dem partiellen de-Bruijn-Graphen;
Berechnen der Erstreckungsstärke in der Richtung der Erstreckung für jeden Verzweigungsvertex in einer zuerst in der Tiefe angewandten Art und Weise in eine Richtung, und Erzeugen einer neuen Kante und eines neuen Verzweigungsvertexes nach jeder Berechnung aus den Basenerstreckungen, die eine Erstreckungsstärke oberhalb eines Schwellwertes aufweisen;
falls es in einem Pfad keine Basenerstreckungen gibt, die eine Erstreckungsstärke oberhalb des Schwellwertes aufweisen, Rückgabe eines Fehlers für den Pfad; und
falls ein neuer Verzweigungsvertex erzeugt wird, der gleich der Basensequenz eines der Referenzvertexe ist und der konsistent ist mit einem SNP oder einem kurzen Indel, Beenden der Berechnung und Rückgabe des Pfades.

11. Verfahren nach Anspruch 1, wobei das Kombinieren der lokalen Bereiche und der lokalen de-novo-Intervalle zum Bilden von Optimierungsintervallen beinhaltet:

Berücksichtigen, als Kandidatenoptimierungsintervalle, der lokalen de-no-vo-Intervalle und der Referenzwerte, die mit einem hohen Wahrscheinlichkeitsverhältnis $P_V/P_{Ref}$, das den Schwellwert überschreitet, assoziiert sind, wobei $P_V$ die Wahrscheinlichkeit einer 1-Basenvariationshypothese ist, und wobei $P_{Ref}$ eine Wahrscheinlichkeit des Basenwertes in der Referenzpolynucleotidsequenz ist; und
Kombinieren der Kandidatenoptimierungsintervalle, die überlappen oder weniger als einen Schwellwert eines Basenabstands voneinander entfernt sind, in die Optimierungsintervalle;

wobei die Probenpolynucleotidsequenz ein Genom $G$ umfasst, und wobei jeder der Referenzwerte ein logarithmisches Wahrscheinlichkeitsverhältnis $L(G)$ für jede der Hypothesen umfasst, wobei $L(G) = \log(P_V/P_{Ref})$.

12. Verfahren nach Anspruch 1, wobei das Modifizieren der initialen Sequenzhypothese, um eine optimierte Sequenzhypothese zu erhalten, umfasst:

Durchlaufen jeder Basenposition in einer initialen Hypothese in dem Optimierungsintervall und iteratives Ändern der Base mit allen möglichen alternativen Basenwerten einschließlich eingefügter und gelöschter Basen, wobei für jede Änderung ein Wahrscheinlichkeitsverhältnis berechnet wird; und
Anwenden von Änderungen auf die initialen Hypothesen, die das Wahrscheinlichkeitsverhältnis maximieren.

13. System, umfassend:

einen Datenspeicher (14), der eine Referenzpolynucleotidsequenz sowie gemappte gepaarte Ablesungen speichert, die von einer Probenpolynucleotidsequenz erhalten wurden, und auf Orte in der Referenzpolynucleotidsequenz gemappt sind;

ein Computercluster (10) umfassend eine Mehrzahl von Computern (12), die mit dem Datenspeicher über ein Netzwerk verbunden sind; und

einen Variationenaufrufer (18), der parallel auf der Mehrzahl von Computern ausgeführt wird, wobei der Variationenaufrufer ausgebildet ist, das Verfahren nach einem der Ansprüche 1-12 auszuführen.

**14.** System nach Anspruch 13, wobei das Computercluster so ausgebildet ist, dass Instanzen des Variationenaufrufers, die auf unterschiedlichen Computern der Mehrzahl von Computern ausgeführt werden, auf unterschiedlichen Abschnitten der Referenzpolynucleotidsequenz und der gemappten gepaarten Ablesungen parallel arbeiten.

**15.** Ausführbares Softwareprodukt, das auf einem computerlesbaren Medium gespeichert ist und Programbefehle zum Ausführen des Verfahrens nach einem der Ansprüche 1-12 enthält.

## Revendications

**1.** Procédé mis en oeuvre par ordinateur pour appeler des variations dans des lectures appariées cartographiées qui ont été obtenues à partir d'une séquence polynucléotidique d'échantillon par comparaison à une séquence polynucléotidique de référence, le procédé comprenant:

recevoir la séquence polynucléotidique de référence et les lectures appariées cartographiées (200), dans lequel les lectures appariées cartographiées sont obtenues de la séquence polynucléotidique d'échantillon et sont cartographiées sur des emplacements dans la séquence polynucléotidique de référence;
pour chacune d'une pluralité de positions sur la séquence polynucléotidique de référence:

calculer une ou plusieurs valeurs de référence (510), chaque valeur de référence appartenant à une hypothèse qui est différente de la séquence polynucléotidique de référence sur la position et qui est calculée sur la base des lectures appariées cartographiées en utilisant une formulation bayésienne (500) pour l'hypothèse;

identifier des zones locales (300) correspondant à des positions ayant une valeur de référence supérieure à une valeur de seuil;
utiliser des algorithmes basés sur des graphes de de Brujin afin de déterminer des graphes pour identifier des intervalles de novo locaux (512), chaque intervalle de novo local comprenant une ou plusieurs positions où le graphe diffère de la séquence polynucléotidique de référence;
combiner les zones locales et les intervalles de novo locaux afin de former des intervalles d'optimisation (514);
pour chaque intervalle d'optimisation:

utiliser les valeurs de référence ou un ou plusieurs graphes de l'intervalle d'optimisation pour générer une hypothèse initiale de séquence (412); et
modifier l'hypothèse initiale de séquence afin d'obtenir une hypothèse optimisée de séquence (414) présentant une probabilité accrue d'être correcte, sur la base des lectures appariées cartographiées qui cartographient sur l'intervalle d'optimisation;

utiliser les hypothèses de séquence optimisées pour identifier et appeler des variations (32) qui sont détectées dans les lectures appariées cartographiées en relation à la séquence polynucléotidique de référence; et
délivrer une liste des variations, chacune décrivant une manière dont les lectures appariées cartographiées sont observées comme étant différentes de la séquence polynucléotidique de référence sur ou à proximité d'un emplacement déterminé.

**2.** Procédé selon la revendication 1, dans lequel l'action de délivrer la liste des variations comprend en outre l'action de délivrer une liste de régions non appelées pour lesquelles des variations ne peuvent pas être appelées en raison d'incertitudes de calcul.

**3.** Procédé selon la revendication 1, dans lequel les variations comprennent des séquences identifiées de délétions, d'insertions, de mutations, de polymorphismes, et de duplications ou de réarrangements d'une ou de plusieurs bases.

4. Procédé selon la revendication 3, comprenant en outre une utilisation des hypothèses de séquence optimisées afin d'assembler la séquence polynucléotidique d'échantillon à partir des lectures appariées cartographiées, dans lequel une séquence polynucléotidique assemblée est basée pour l'essentiel sur la séquence polynucléotidique de référence, mais comprend les séquences identifiées.

5. Procédé selon la revendication 1, dans lequel chacune des lectures appariées cartographiées comprend des lectures espacées de manière variable.

6. Procédé selon la revendication 1, dans lequel chacune des lectures appariées cartographiées comprend des lectures non espacées.

7. Procédé selon la revendication 1, dans lequel le calcul de valeurs de référence en utilisant la formulation bayésienne comprend:

   pour chaque position de base dans la séquence polynucléotidique de référence:

   générer un jeu d'hypothèses initiales pour cette position de base dans la séquence polynucléotidique de référence en modifiant une valeur de base sur cette position de base dans p allèles par toutes les variations de base 1 possibles;
   déterminer un jeu de lectures appariées cartographiées qui sont proches de cette position de base de la séquence polynucléotidique de référence; et
   calculer des valeurs de référence pour cette position de base en calculant un rapport de probabilité $P_v/P_{Ref}$ pour chacune des hypothèses initiales dans ledit jeu d'hypothèses initiales, dans lequel $P_v$ est une probabilité d'une hypothèse de variation de base 1, et $P_{Ref}$ est une probabilité de la valeur de base dans la séquence polynucléotidique de référence, et dans lequel le jeu de lectures appariées cartographiées proches de cette position de base est utilisé durant le calcul du rapport de probabilité sur cette position de base;

   dans lequel la séquence polynucléotidique d'échantillon comprend un génome $G$, et dans lequel chacune des valeurs de référence comprend un rapport de probabilité logarithmique $L(G)$ pour chacune des hypothèses, où $L(G)$ = log $(P_v/P_{Ref})$.

8. Procédé selon la revendication 6, dans lequel les lectures appariées cartographiées sont générées indépendamment les unes des autres, et dans lequel des estimations de probabilité qui prennent en compte l'ensemble des lectures appariées cartographiées sont calculées par:

$$\frac{P(G|MtdRds)}{P(G_0|MtdRds)} = \left(\frac{N_{G_0}}{N_G}\right)^{N_D} \prod_{MtdRds} \frac{\sum_M P(g)P(MtdRd|G,M)}{\sum_M P(g)P(MtdRd|G_0,M)}$$

où $N_{G_0}$ représente un nombre de bases dans le génome de référence, $N_G$ représente un nombre de bases dans le génome d'échantillon et $N_D$ représente un nombre de lectures appariées.

9. Procédé selon la revendication 7, comprenant en outre une représentation de $\left(\frac{N_{G_0}}{N_G}\right)^{N_D}$ avec une approximation de pénalité d'insertion de sorte que chaque base supplémentaire dans un allèle de $G$ provoque une diminution dans $P(G|MtdRds)$ par un facteur exp$(-c/n_D)$, dans lequel $n_D$ représente un nombre de bases dans chacune des lectures appariées cartographiées de sorte que des bases supplémentaires ne sont pas additionnées à $G$, sauf que les bases supplémentaires ont un support suffisant par des lectures appariées cartographiées, où c est la couverture moyenne par allèle.

10. Procédé selon la revendication 1, dans lequel le calcul d'intervalles de novo locaux utilise un graphe de de Brujin partiel afin de trouver des variations au-delà de changements de base individuels, le procédé comprenant en outre:

    initialiser le graphe de de Brujin partiel avec des vertex de référence créés à partir de séquences de base provenant de la séquence polynucléotidique de référence;
    déterminer, pour chacun des vertex de référence, un jeu de lectures appariées cartographiées qui cartographient sur le vertex de référence et qui comprennent une extension de base qui s'étend au-delà de l'une des deux extrémités du vertex de référence par toute valeur de base 1 possible;

calculer, pour chacune des extensions de base, une intensité d'extension représentant une valeur de support pour étendre le vertex de référence par chaque valeur de base 1, sur la base, au moins en partie, d'un nombre de lectures appariées cartographiées présentant la même extension, et du nombre de concordances et de non-concordances de ces lectures appariées cartographiées avec la séquence du vertex qui est traité;

utiliser les extensions de base ayant l'intensité d'extension la plus importante incompatible avec les vertex de référence, en tant que vertex de ramification dans le graphe de de Brujin partiel;

calculer l'intensité d'extension dans la direction de l'extension pour chaque vertex de ramification d'une manière appliquée d'abord dans la profondeur, dans une direction, et créer une nouvelle arête et un nouveau vertex de ramification après chaque calcul à partir des extensions de base présentant une intensité d'extension supérieure à une valeur de seuil;

s'il n'y a pas d'extensions de base sur un trajet, qui présentent une intensité d'extension supérieure à la valeur de seuil, retourner un défaut pour le trajet; et,

si un nouveau vertex de ramification est créé qui est égal à la séquence de base de l'un des vertex de référence et qui est consistent avec un SNP ou un indel court, terminer le calcul et retourner le trajet.

11. Procédé selon la revendication 1, dans lequel la combinaison des zones locales et des intervalles de novo locaux pour former des intervalles d'optimisation comprend:

prendre en considération, en tant qu'intervalles d'optimisation candidats, les intervalles de novo locaux et les valeurs de référence qui sont associés avec un rapport élevé de probabilité $P_v/P_{Ref}$ qui dépasse la valeur de seuil, où $P_v$ est la probabilité d'une hypothèse de variation de base 1, et où Prof est une probabilité de la valeur de base dans la séquence polynucléotidique de référence; et

combiner les intervalles d'optimisation candidats qui chevauchent ou sont à moins d'une valeur de seuil d'une distance de base les uns des autres, dans les intervalles d'optimisation;

dans lequel la séquence polynucléotidique d'échantillon comprend un génome G, et dans lequel chacune des valeurs de référence comprend un rapport de probabilité logarithmique L(G) pour chacune des hypothèses, où $L(G)$ = log $(P_v)$ $/P_{Ref}$.

12. Procédé selon la revendication 1, dans lequel la modification de l'hypothèse initiale de séquence pour obtenir une hypothèse optimisée de séquence comprend:

passer à travers chaque position de base dans une hypothèse initiale dans l'intervalle d'optimisation et changer de manière itérative la base avec toutes les valeurs de base alternatives possibles, y compris les bases insérées et supprimées, un rapport de probabilité étant calculé pour chaque changement; et

appliquer des changements aux hypothèses initiales qui maximisent le rapport de probabilité.

13. Système comprenant:

une mémoire de données (14) qui mémorise une séquence polynucléotidique de référence ainsi que des lectures appariées cartographiées obtenues à partir d'une séquence polynucléotidique d'échantillon et cartographiées sur des emplacements dans la séquence polynucléotidique de référence;

une grappe d'ordinateurs (10) comprenant une pluralité d'ordinateurs (12) qui sont connectés à ladite mémoire de données par un réseau; et

un programme d'appel de variations (18) exécuté en parallèle sur ladite pluralité d'ordinateurs, ledit programme d'appel de variations étant configuré pour mettre en oeuvre le procédé selon l'une quelconque des revendications 1 à 12.

14. Système selon la revendication 13, dans lequel ladite grappe d'ordinateurs est configurée de telle sorte que des instances du programme d'appel de variations qui sont exécutées sur des ordinateurs différents de ladite pluralité d'ordinateurs travaillent en parallèle sur des portions différentes de la séquence polynucléotidique de référence et des lectures appariées cartographiées.

15. Produit logiciel exécutable qui est mémorisé sur un support lisible par ordinateur et comprend des instructions de programme pour mettre en oeuvre le procédé selon l'une quelconque des revendications 1 à 12.

# FIG. 1

Mated Read 200

Left Arm 210A | Right Arm 210B

Reads 204

r8  r7  r6  r5  Mate Gap 208  r4  r3  r2  r1
                (g3)
g5        g4        g2        g1

Gaps 206

Base Reads 202

| C | T | C | T | C | G | C | C | A | G |

97 82 92 93 90 90 86 71 43 90 ← Quality Score 214

## FIG. 2

Reference Polynucleotide Sequence (G₀) 24       N_G0 Total number of bases

Local Area 300

Mate Gap 208

Mapped Mated Reads 28

## FIG. 3

Execute an application on at least one computer that locates local areas in the reference polynucleotide sequence where a likelihood exists that one or more bases of the sample polynucleotide sequence are changed from corresponding bases in the reference polynucleotide sequence, where the likelihood is determined at least in part based on mapped mated reads of the sample polynucleotide sequence
400

Generate at least one sequence hypothesis for each of the local areas and optimize the at least one sequence hypothesis for at least a portion of the local areas to find one or more optimized sequence hypotheses of high probability for the local areas
402

Analyze the optimized sequence hypotheses to identify a series of variation calls in the sample polynucleotide sequence
404

# FIG. 4A

FIG. 4B

FIG. 4C

Optimized Sequence
Hypotheses 414A

Variation Calls 32

Optimized Sequence
Hypotheses 414A

# FIG. 4D

FIG. 5

Generate a set of initial hypotheses for each base position in the reference $G_0$ by modifying the base value at that position in $p$ alleles by all possible 1-base variations
600

Determine a set of mapped mated reads that are near the current base position of the reference $G_0$
602

Compute reference scores for each base position by computing for each of the initial hypotheses in the corresponding set, a probability $P_v/P_{ref}$, where $P_v$ is a probability of a 1-base variation hypothesis, and $P_{ref}$ is a probability of the base value in the reference $G_0$, and where the set of mapped mated reads near each base position are used during calculation of the probability ratio at each base position
604

Compute Reference Scores using
Bayesian Formulation

FIG. 6

Initialize de Bruijn graph with reference vertices, where each of the reference vertices represent a base sequence of N contiguous bases from the reference
700

For each of the reference vertices, determine a set of mapped reads that map to the reference vertex and that include a base extension extending beyond either end of the reference vertex in a left or right direction by any possible base value, and calculate for each base extension a base extension strength representing an amount of support for extending the reference vertex by each 1-base value, and indicate a left or right direction of the base extension
702

Partition the reference polynucleotide sequence into reference segments
704

Find likely variations from the reference by finding M top base extensions incompatible with the reference vertices in each of the reference segments and whose base extension strength is greater than a first threshold
706

Use the M top base extensions as branch vertices in a partial de Bruijn graph and input each branch vertex and direction of extension into a recursive procedure that builds the partial de Bruijn graph
708

(A)

Compute local de novo intervals
using a partial de Bruijn procedure

# FIG. 7A

Ⓐ

In a depth-first recursive process, determine a set of mapped reads that map to the branch vertex and that includes a base extension that extends beyond the branch vertex in the corresponding direction by each possible base value (A C G or T/U), and calculate for each base extension a branch extension strength representing an amount of support for extending the branch vertex by each base value
710

Create new branch vertices from the base extensions having the second extension strength above a branch extension threshold
712

Construct an edge between the branch vertex and each of the new branch vertices, wherein a chain of the branch vertices connected by one or more edges forms a path through the graph
714

Calculate an edge extension strength for the edges in the path, and find the top N edges with the largest edge extension strength above an edge strength threshold, and perform block 722 using each of the top N edges
716

If block 722 fails to find a match between the new branch vertices and the reference vertices, then the top N new edges are input into the recursive procedure as branch vertices
718

Y

N

If no edges have an edge extension strength above the edge strength threshold, end the recursive procedure and return failure for the path
720

Compare each of the new branch vertices to the reference vertices in the partial de Bruijn graph that represent nearby base sequences from the reference, and if the new branch vertex equals any of the base sequences of the reference vertices, end the recursive procedure and return the path
722

# FIG. 7B

Analyze the reference score associated with each reference base position and identify as a peak each range of consecutive positions at which the reference scores are above a predetermined magnitude threshold, i.e., high L(G)
800

For each identified peak, define as a peak interval a set of consecutive base positions formed by the base position of the peak and a predefined number of bases on either side of that base position
802

Form respective candidate optimization intervals from each of the peak intervals, and each of the local de novo intervals in branches of the Bruijn graph
804

Create a union of the candidate optimization intervals that overlap
806

Merge neighboring candidate optimization intervals that are less than a prescribed distance apart
808

Assign a no call to the candidate optimization intervals that are longer than a predetermined base length, and add to a list of no-call intervals
810

Add remaining candidate optimization intervals to a list of optimization intervals
812

Finding Optimization Intervals

FIG. 8

Fetch an optimization interval from the set of
optimization intervals and set as active interval
900

Determine a set of mapped mated reads that are
near the active interval
902

Construct a de Bruijn Graph using a portion of
reference $G_0$ containg the active interval and
mapped mated reads for the active interval
904

Fetch a path of sequences in the de Bruijn Graph
that rejoin the reference path in the graph for use
as a starting point for sequence hypotheses
906

Optimize the sequence hypotheses by applying to
the current p alleles of G all possible 1- base
changes only in the active interval
908

For each modified G, compute L(G) using mapped
mated reads near active interval
910

Apply to G 1-base changes that result in the
largest L(G)
912

L(G)
improved?
914

Y

N

(A)

Optimization using a
Bayesian Formulation
and a de Bruijn Graph

# FIG. 9A

(B)  (C)

Ⓐ                                      Ⓑ  Ⓒ

Store details for all sequence hypotheses for G
that have a computed probability within a given
factor of the most likely hypothesis found, up to a
maximum number of hypotheses
916

More
Paths?        Y
918

N

More Opt.      Y
Intervals?
920

N

End

# FIG. 9B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20090111705 A
- US 20090111706 A
- US 20090075343 A
- US 6864052 B
- US 6309824 B
- US 6401267 B
- US 6210891 B
- US 6828100 B
- US 6833246 B
- US 69113457329496 A
- US 6306597 B
- WO 2006073504 A
- WO 2007120208 A
- US 5795782 A

- US 6015714 A
- US 6627067 B
- US 7238485 B
- US 7258838 B
- US 2006003171 A
- US 20090029477 A
- US 20090111115 A
- US 20080234136 A
- US 20070099208 A
- WO 61149670 A
- WO 61149665 A
- WO 61149689 A
- EP 1216524731225 A
- US 61173967 B

### Non-patent literature cited in the description

- **HENG L. et al.** Mapping short DNA sequencing reads and calling variants using mapping quality scores. *Genome Research,* 2008, vol. 18 (11), 1851-1858
- **MARTH G. T. et al.** A general approach to single-nucleotide polymorphism discovery. *Nature Genetics,* 1999, vol. 23 (4), 452-456
- Genome Analysis: A Laboratory Manual Series. 1999, vol. I-IV
- Genetic Variation: A Laboratory Manual. 2007
- PCR Primer: A Laborafory Manual. 2003
- **BOWTELL ; SAMBROOK.** DNA Microarrays: A Molecular Cloning Manual. 2003
- **MOUNT.** Bioinformafics: Sequence and Genome Analysis. 2004
- **SAMBROOK ; RUSSELL.** Condensed Protocols from Molecular Cloning: A Laboratory Manual. 2006

- **SAMBROOK ; RUSSELL.** Molecular Cloning: A Laboratory Manual. Cold Spring Harbor Laboratory Press, 2002
- **STRYER, L.** Biochemistry. W.H. Freeman, 1995
- **GAIT.** Oligonucleoride Synthesis: A Practical Approach. IRL Press, 1984
- **NELSON ; COX.** Lehninger, Principles of Biochemistry. W. H. Freeman Pub, 2000
- **BERG et al.** Biochemistry. W.H. Freeman Pub, 2002
- **MARGULIES et al.** *Nature,* 2005, vol. 437, 376-380
- **RONAGHI et al.** *Anal. Biochem.,* 1996, vol. 242, 84-89
- **MARTH GT et al.** *Nature Genetics,* 1999, vol. 23, 452-456